# EUROPEAN PATENT SPECIFICATION

(11) **EP 3 589 924 B1**
(45) Date of publication and mention of the grant of the patent: **08.01.2025**
(21) Application number: 18707962.9
(22) Date of filing: 23.02.2018
(51) Int. Cl.: G01F 11/26, A47G 19/22, A47G 23/16

(54) **DRINKING APPARATUS, SYSTEM AND ASSOCIATED METHODS**
TRINKVORRICHTUNG, SYSTEM UND ZUGEHÖRIGE VERFAHREN
APPAREIL À BOISSON, SYSTÈME, ET PROCÉDÉS ASSOCIÉS

(30) Priority: 02.03.2017 GB 201703379
(43) Date of publication of application: 08.01.2020
(73) Proprietor: Spearmark Holdings Limited, Eaton Socon St Neots Cambridgeshire PE19 8GA (GB)
(72) Inventor: BLOOM, Terry, St Neots Cambridgeshire PE19 8GA (GB); VAN LEEUWEN, Eleanor, Maidstone Kent ME17 3QL (GB); CUNNINGHAM, Arthur, Hempstead Hertfordshire HP1 1RP (GB)
(74) Representative: Boult Wade Tennant LLP
(86) International application number: PCT/GB2018/050482
(87) International publication number: WO 2018/158561

(56) References cited:
- WO-A1-2013/059579
- WO-A1-2015/024575
- CN-A- 105 520 838
- US-A1- 2015 122 688

## Description

The present disclosure relates to a drinking apparatus, a system and associated methods. In particular associated with the aim of reminding a user to take a drink at intermittent times.

### Background to the Disclosure

Adequate hydration is an important part of general well-being. In some contexts and for some people maintaining adequate hydration is more difficult. For example, patients in a care setting, e.g. bed-ridden, in a nursing home, or in a hospital, may find it difficult to remember to regularly take drinks and may have difficulty accessing water at regular intervals. For some patients, it may be useful to provide a reminder for the patient to take a drink at intermittent times.

US2015122688 relates to a retainer, a lid, and a sensor, where the sensor is configured to detect information about the retainer, the lid, or the contents in the retainer. The sensor also may be configured to communicate with an internal or external computer system, thereby facilitating showing the detected information as a representation via a display element. The system may include an action element such as an open/close lid opening assembly configured to permit automatically or manually opening or closing a drink aperture or another type of dispensing aperture.

WO2015024575 relates to a device arranged to be used as a cup or a cup holder for liquid or medicaments and/or for receiving a cup for liquid or medicaments comprising: a receptacle for storing said liquid, medicament or cup. The device further comprises a time assessment device, a motion sensor, and communication means. WO2015024575 further relates to a method to alert a cup or cup holder user that the cup or cup holder has not been used for a certain time period. The method comprises the steps of: starting said time assessment device, checking if said motion sensor has detected motion, and resetting the time assessment device in the case of detected motion, triggering the communication means to communicate when the time assessment device has reached a first pre-determined time assessment.

GB2499829 provides a drinking aid for reminding a user to take a drink. However, the drinking aid is not particularly suitable for use in a care setting as it would be prone to trapping germs and would not allow adequate infection control. Also, the drinking aid is not adapted for a particular setting or for use by particular users.

According to WO2013059579 a light-emitting container such as a bottle includes a hollow vessel and a light-emitting device. The vessel has an open end and a closed end. At least a portion of the vessel is one of transparent and translucent. The light-emitting device is disposed adjacent the closed end of the vessel. The light-emitting device includes a microcontroller in electrical communication with at least one light source. The microcontroller selectively causes one of an activation and a deactivation of the at least one light source. A light emitted from the at least one light source is transmitted through the vessel upon the activation of the at least one light source.

Consequently, it is an object of the present disclosure to provide an improved drinking apparatus, system and method. The improved drinking apparatus, system and method may be more suitable for use in a care setting and provide more helpful methods of reminding a user to take a drink at intermittent times. They may in addition be more suitable for use in situations where infection control regimes are in operation.

### Summary of the Disclosure

Accordingly, in a first aspect the present disclosure provides a drinking apparatus with the features of claim 1, comprising a receptacle and a sensor module;
the receptacle defining a cavity for holding a liquid for consumption which can be filled into the cavity and drained from the cavity through a mouth of the receptacle;
wherein the sensor module can be coupled to the receptacle;
wherein the sensor module can also be decoupled from the receptacle to allow the receptacle to be cleaned separately from the sensor module;
the drinking apparatus being able to freely stand on a support surface in a first, storage, orientation in which liquid can be retained in the cavity;
the sensor module comprising an alert system which is able, when the sensor module is coupled to the receptacle, to detect a reorientation of the drinking apparatus into a second, cleaning, orientation in which the mouth of the receptacle is angled downwards;
wherein the sensor module further comprises a reminder system to intermittently prompt a user to consume liquid from the receptacle,
wherein the reminder system comprises:
- one or more sensors configured to detect a drinking event associated with a user consuming liquid from the cavity;
- a timer configured to measure one or more predetermined time intervals;
- a speaker configured to generate one or more audio alerts and/or one or more lights configured to generate one or more visual alerts;
- a controller configured to generate a user alert if no drinking event is detected by the one or more sensors within a selected predetermined time interval as measured by the timer;
- a control interface; and
- a power source; wherein the user alert is one or more visual alerts generated by the one or more lights and/or one or more audio alerts generated by the speaker; wherein the control interface is configured to permit the user of the drinking apparatus to operate functions of the drinking apparatus; and whereby the alert system is configured to generate a warning signal on detection of a reorientation of the drinking apparatus into the second, cleaning, orientation to warn a user against cleaning the drinking apparatus while the sensor module remains coupled to the receptacle; wherein the controller is configured to reset the timer and restart measurement of the one or more predetermined time intervals whenever a drinking event is detected.

Advantageously, the drinking apparatus of the present disclosure helps to prevent a user inadvertently exposing the sensor module of the drinking apparatus to a cleaning environment that may damage the sensor module. For example, the cleaning environment may be a source of water such as a running tap, a sink, or a dishwasher. A particular source of concern may be exposing the sensor module to the cleaning environment of a dishwasher due to the high temperatures, salt and cleaning agents typically used in such devices. The risk of inadvertent exposure to such cleaning environments is reduced by use of the alert system that detects when the drinking apparatus (including the coupled sensor module) is reorientated into the second, cleaning orientation. This is because typically a user wishing to clean the drinking apparatus will need to wholly or partially invert the receptacle. For example, in a dishwasher a user would wish to place the drinking apparatus in an upturned orientation in a rack of the dishwasher wherein the mouth of the receptacle is directed vertically, or nearly vertically downwards. Beneficially, as soon as the user inverts the drinking apparatus to place it in the rack the alert system will be triggered to generate the warning signal reminding the user to decouple the sensor module and thereafter only clean the receptacle in the dishwasher.

The term "warning signal" is used to refer to one or more signals (which may differ in their characteristics from one another) that are generated to be received by the user of the drinking apparatus. For example, the warning signal may be one or more of audio signal(s), visual signal(s) or tactile signal(s).

In this and other aspects of the present disclosure, the liquid for consumption may be, as non-limiting examples, water, a beverage or other preparation whether or not they also include solid elements, e.g. soups, tea, coffee, hot chocolate, etc. The term 'liquid' also encompass gels, fluids and pasty substances that may be consumed in a drinkable manner, for example milkshakes, high-fibre supplements, ice slushes, etc. In one preferred use, the drinking apparatus of the present disclosure finds application as a water container for drinking water. The drinking apparatus may form a hydration apparatus for a patient. The drinking apparatus is preferably sized for individual use and to be lifted by one hand. The user of the drinking apparatus may be a variety of classes of people. However, the present drinking apparatus provides particular benefits in a healthcare setting where a first class of user may be a patient. A second class of user may be a care-giver that has responsibility for ensuring adequate hydration of the patient and/or cleaning of the drinking apparatus. Both the patient and the care-giver may be users of the same drinking apparatus.

In the second, cleaning, orientation the mouth of the receptacle may be angled downwards to allow liquid to fully, or substantially fully, drain from the cavity out of the mouth of the receptacle. Advantageously, this may allow for all or substantially all of the liquid that might be in the cavity (or enter the cavity during cleaning) to drain under the action of gravity out of the cavity to allow for better cleaning and drying of the receptacle. For example, when the dispensing apparatus is cleaned in a dishwasher it is preferable to orientate the mouth of the receptacle to be angled downwards to prevent the pooling and retention of cleaning water in the cavity during the cleaning cycle of the dishwasher.

In the second, cleaning, orientation the receptacle may have been tilted away from the first, storage, orientation by greater than 90°, preferably greater than 100°, more preferably greater than 135°. For receptacles of differing shapes it may be necessary to tilt the receptacle by a differing angle to reach a suitable cleaning orientation in which the liquid can adequately drain from the mouth. This may depend on the shape of the receptacle, the angle of the side walls of the receptacle, etc. It may be preferred to select a detection angle for the alert system at which the warning signal is generated that is greater than a tilt angle that would be expected to be experienced during normal drinking from the receptacle. This would help reduce the likelihood of triggering the warning signal when drinking from the drinking apparatus. However, this is not essential, only preferred.

In the second, cleaning, orientation the drinking apparatus may be inverted, preferably so that the mouth of the receptacle is directed vertically, or nearly vertically downwards. This would be a typical orientation for the drinking apparatus when placed in an upper rack of a dishwasher (where the platens of the rack are typically angled at a small offset from horizontal to help prevent pooling of cleaning water on the bases of receptacles).

The alert system is configured to generate the warning signal immediately on detection of the reorientation of the drinking apparatus into the second, cleaning, orientation. This may provide the most immediate warning to the user to remove the sensor module, for example before closing of a door of the dishwasher has taken place.

The alert system comprises a sensor configured to detect reorientation of the drinking apparatus into the second, cleaning, orientation.

The sensor may comprise a tilt sensor. The sensor may comprises a 'ball-in-can' tilt sensor. For example, 'ball-in-can' sensor may comprises a cylindrical housing (can) provided with two spaced apart switch contacts at one end of the housing. Within the housing is provided one or more electrically-conductive balls that can move between one end of the housing away from the switch contacts to the other end of the housing where the ball(s) bridge between the switch contacts closing the circuit. Movement of the ball(s) within the can is triggered by tilting of the housing.

As an alternative to a tilt sensor the sensor may comprise a gyroscope or hall effect sensor. More than one sensor may be used in combination to detect reorientation of the drinking apparatus.

The alert system may comprise one or more lights configured to generate the warning signal in the form of a visual alert. The one or more lights may comprise one or more light-emitting diodes (LEDs). The one or more lights may be located within an interior of the sensor module and may be configured to transmit light through a face of the sensor module. The visual alert may comprise one or more patterns of intermittent flashing or pulsing of the one or more lights.

The alert system may comprise a speaker configured to generate the warning signal in the form of an audible alert. The speaker may be located within an interior of the sensor module and may be configured to transmit sound waves via a face of the sensor module; and wherein optionally the speaker may be an exciter speaker. Advantageously, the use of an exciter speaker may avoid the need to include any apertures in the sensor module outermost surface since the sound waves are transmitted via the excitation of the surface of the sensor module and not via movement of air through apertures. The absence of such apertures in the sensor module helps to avoid the creation of cavities in the outer surface of the sensor module which might otherwise provide a location for breeding of germs.

Alternatively, a portion of a housing of the sensor module may be provided with an array of apertures for transmission of sound waves. A separate thin membrane may then be affixed to an exterior of the housing of the sensor module to cover the apertures. The membrane may be secured to the housing around the circumferential periphery only to allow the membrane to resonate freely against the housing when driven by sound waves produced by a speaker located within the interior of the sensor module. The membrane may be formed from a plastics material that may be flexible. Advantageously, the apertures may improve the transmission of the sound waves out of the sensor module while the membrane helps to prevent the creation of any cavities on the exterior of the sensor module which might otherwise provide a location for breeding of germs. Protrusions may be provided on the housing exterior to create a space between the membrane and a support surface which may provide space for the membrane to resonate freely.

The alert system may comprise a tactile signal generator. For example, the alert system may comprise a vibrator that causes vibration of the sensor module.

The alert system may comprise any combination of the above-described lights, speakers and/or tactile signal generators.

The sensor module and the receptacle may comprise complementary connection means to permit coupling and decoupling of the sensor module and the receptacle; optionally wherein the complementary connection means comprises screw threads.

The receptacle may comprise a recess which receives at least a portion of the sensor module on coupling of the sensor module to the receptacle.

A seal may be provided between the sensor module and the receptacle to hinder or prevent ingress of water there between.

At least a portion of the sensor module may be formed from a transparent or translucent material.

The sensor module may be coupled to any suitable portion of the receptacle. However, it may be particularly beneficial where the sensor module is coupled to the receptacle below the cavity. In this way the centre of gravity of the drinking apparatus may be lowered by locating the sensor module towards the bottom/base of the drinking apparatus. This may improve the stability of the drinking apparatus. In some embodiments the sensor module may form a base unit of the drinking apparatus. The drinking apparatus may be able to freely stand on the support surface with the base unit in contact with the support surface. The sensor module comprises a reminder system to intermittently prompt a user to consume liquid from the receptacle. The reminder system comprises one or more sensors configured to detect a drinking event associated with a user consuming liquid from the cavity. The one or more sensors configured to detect a drinking event associated with a user consuming liquid from the cavity may comprise a sensor configured to detect tilting of the receptacle away from the vertical; and preferably the sensor may be configured to detect tilting of the receptacle away from the vertical by greater than 15°, preferably greater than 30°. The sensor configured to detect tilting of the receptacle away from the vertical may comprise a tilt sensor. As an alternative to a tilt sensor the sensor may comprise a gyroscope or hall effect sensor.

The drinking apparatus comprises the sensor configured to detect reorientation of the drinking apparatus into the second, cleaning, orientation and a separate one or more sensors configured to detect a drinking event associated with a user consuming liquid from the cavity. Advantageously, this may allow for the performance characteristics of each sensor to be adapted for the different requirements of detecting tilting associated with drinking and detecting a tilting (typically greater tilting) associated with inverting the drinking apparatus into the second, cleaning, orientation. For example, different designs of tilt sensor may be used for the different sensors.

The reminder system further comprises:
- a timer configured to measure one or more predetermined time intervals;
- a speaker configured to generate one or more audio alerts and/or one or more lights configured to generate one or more visual alerts;
- a controller configured to generate a user alert if no drinking event is detected by the one or more sensors within a selected predetermined time interval as measured by the timer;
- a control interface; and
- a power source;

wherein the user alert is one or more visual alerts generated by the one or more lights and/or one or more audio alerts generated by the speaker;
wherein the control interface may be configured to permit the user of the drinking apparatus to operate functions of the drinking apparatus.

The control interface may be configured to permit the user of the drinking apparatus to:
- select one or more predetermined time intervals to be used by the controller for generation of the user alert; and
- select the user alert from the one or more visual alerts and/or the one or more audio alerts.

The reminder system may further comprise:
- an audio recorder configured to allow recording of one or more audio messages by a user of the drinking apparatus - said one or more audio messages then being used as the one or more audio alerts;
and optionally the control interface may be configured to permit the user of the drinking apparatus to record the one or more audio messages.

When the sensor module is coupled to the receptacle the control interface may be inaccessible by the user; and optionally the control interface may comprise a plurality of buttons for inputting user commands which may be inaccessible when the sensor module is coupled to the receptacle. Advantageously, this helps to prevent accidental activation of the control interface when a user is drinking from the drinking apparatus. In addition, where the drinking apparatus is for a patient who is under the supervision of a care-giver, the control interface may be operated by the care-giver and the patient may be hindered from accidentally or deliberately altering the setting of the drinking apparatus during use.

The receptacle may comprise a recess which may receive at least the control interface of the sensor module on coupling of the sensor module to the receptacle so as to render the control interface inaccessible to the user while the sensor module remains coupled to the receptacle.

The power source may comprise one or more batteries housed in a battery compartment, and when the sensor module is coupled to the receptacle the battery compartment may be inaccessible by the user.

The drinking apparatus may further comprise an ambient light sensor for detecting an ambient light level around the drinking apparatus when on the support surface. The ambient light sensor may be housed within an interior of the sensor module and may be configured to detect the ambient light level by detecting signals transmitted through a face of the sensor module.

Advantageously, the one or more sensors configured to detect a drinking event and the ambient light sensor do not require any apertures in the sensor module to function. This may help to reduce the number of cavities in the sensor module's outer surface that could provide a location for retention and growth of germs. It may also assist in allowing the sensor module to be configured as preferably splash-proof and/or water-resistant.

The controller may be configured to provide a night-time mode in which generation of the user alert is prevented whenever the ambient light level is below a predetermined level as detected by the ambient light sensor. Advantageously, this may help to prevent disturbance of the user during periods of sleep, e.g. at night, when drinking is not required. In the night-time mode the controller may be configured to activate the one or more lights when the ambient light level is below a predetermined level as detected by the ambient light sensor to thereby provide illumination for the user to locate the drinking apparatus. Advantageously, this may help a user to locate the drinking apparatus at night if the user wakes and wishes to take a drink. In the night-time mode the one or more lights may be activated in a mode pre-programmed to be one of: constant illumination, pulsing illumination or flashing illumination.

The drinking apparatus may further comprise a microphone located within an interior of the sensor module.

The controller may be configured to use one or more pre-recorded default audio alerts in the eventuality that the one or more audio messages are not recorded or selected. The controller may be configured to use the one or more audio messages sequentially if no drinking event is detected. The control interface may be configured to permit the user of the drinking apparatus to select a volume of the one or more audio alerts. The controller may be configured to repeat each audio alert at an increased volume. The one or more predetermined time intervals may include 20 minutes, 40 minutes and 60 minutes. The controller is configured to reset the timer and restart measurement of the one or more predetermined time intervals whenever a drinking event is detected. The controller may be configured to interrupt the user alert, if being generated, whenever a drinking event is detected.

An interior face of the receptacle may comprise one or more filling-level markings, preferably annular rings.

The drinking apparatus may further comprise a lid that is detachably coupled to an open mouth of the receptacle.

In the present disclosure there is also provided a drinking system comprising a drinking apparatus as described above and further comprising a second receptacle which can be detachably coupled to said sensor module in place of the receptacle. The second receptacle may have a different shape and configuration to the receptacle. For example, one of the receptacles may be configured as a tumbler (not having a projecting handle) and another of the receptacles may be configured as a mug (having a projecting handle). An optional lid may also be provided as part of the drinking system. The lid may be a disposable part of the drinking system.

The present disclosure also relates to the sensor module of the drinking apparatus as described above and in the other aspects The present disclosure also relates to the receptacle of the drinking apparatus as described above and in the other aspects below. This sensor module and this receptacle do not form a part of the current invention.

The present invention in a second aspect relates to a method of preventing damage to a drinking apparatus, wherein the drinking apparatus is as described above in the first aspect, the method comprising the steps of:
using the alert system to detect a reorientation of the drinking apparatus into the second, cleaning, orientation;
on said detection, generating a warning signal to warn a user against cleaning the drinking apparatus while the sensor module remains coupled to the receptacle.

The method may comprise using the alert system to detect when the mouth of the receptacle has been angled downwards to allow liquid to fully, or substantially fully, drain from the cavity out of the mouth of the receptacle, and then generating the warning signal. The method may comprise using the alert system to detect when the receptacle has been tilted away from the first, storage, orientation by greater than 90°, preferably greater than 100°, more preferably greater than 135°, and then generating the warning signal. The method may comprise using the alert system to detect when the drinking apparatus is inverted, preferably so that the mouth of the receptacle is directed vertically, or nearly vertically downwards, and then generating the warning signal. The method may comprise generating the warning signal immediately on detection of the reorientation of the drinking apparatus into the second, cleaning, orientation.

A particular advantage of the drinking apparatus of all of the aspects of the present disclosure is that it may form a modular system that can be adapted for different users' needs. The modular system is formed by the constituent parts of one or more configurations of receptacle, a sensor module and optionally a lid. For example, at a simplest level the system provides a receptacle that, while adapted to be coupled with a sensor module, is able to be used as a standalone receptacle for the consumption of a liquid, e.g. water. This may be appropriate for a user (e.g. a patient) who is able to reliably remember to consume liquid at regular intervals. If the user has good dexterity then the receptacle may be configured as a tumbler without a handle. If a more secure grip by the user is required then a receptacle configured as a mug with a handle may be used. At the next level the system provides the option of a lid that can be received on the receptacle. The lid may be provided with features such as a mouth port and a straw hole, to assist users that may have difficult holding a receptacle steady. At a further level the system allows for the addition of the sensor module for users that require prompting to consume liquid at regular intervals.

For illustration purposes only, since the drinking apparatus of this invention is defined by all of the features of the appended independent claim 1, this disclosure furthermore relates to a drinking apparatus with the following features: There is provided a drinking apparatus comprising a receptacle and a sensor module;
the receptacle defining a cavity for holding a liquid for consumption by a user of the drinking apparatus;
wherein the sensor module can be coupled to the receptacle;
wherein the sensor module can be decoupled from the receptacle;
the drinking apparatus being able to freely stand on a support surface in a first orientation in which liquid can be retained in the cavity;
wherein the sensor module comprises a reminder system to intermittently prompt a user to consume liquid from the receptacle, the reminder system comprising a control interface;
wherein when the sensor module is coupled to the receptacle the control interface is inaccessible by the user.

As noted above, advantageously, this helps to prevent accidental activation of the control interface when a user is drinking from the drinking apparatus. In addition, where the drinking apparatus is for a patient who is under the supervision of a care-giver, the control interface may be operated by the care-giver and the patient may be hindered from accidentally or deliberately altering the setting of the drinking apparatus during use.

The control interface may comprise one or more buttons for inputting user commands which are inaccessible when the sensor module is coupled to the receptacle.

The receptacle may comprise a recess and when the sensor module is coupled to the receptacle the control interface may be wholly located within said recess.

The sensor module and the receptacle may comprise complementary connection means to permit coupling and decoupling of the sensor module and the receptacle; optionally wherein the complementary connection means comprises screw threads.

The sensor module may further comprise:
- an audio recorder configured to allow recording of one or more audio messages by a user of the drinking apparatus - said one or more audio messages then being used as the one or more audio alerts.

The control interface may be configured to permit the user of the drinking apparatus to operate functions of the drinking apparatus including one or more of:
- switching the drinking apparatus on and off;
- the recording of the one or more audio messages;
- selection of one or more predetermined time intervals to be used by the controller for generation of the user alert;
- selection of the user alert from the one or more visual alerts and/or the one or more audio alerts.

For illustration purposes only, since the drinking apparatus of this invention is defined by all of the features of the appended independent claim 1, this disclosure furthermore relates to a drinking apparatus with the following features: There is provided a drinking apparatus comprising a receptacle and a sensor module;
the receptacle defining a cavity for holding a liquid for consumption by a user of the drinking apparatus;
wherein the sensor module can be coupled to the receptacle;
wherein the sensor module can be decoupled from the receptacle;
the drinking apparatus being able to freely stand on a support surface in a first orientation in which liquid can be retained in the cavity;
wherein the sensor module comprises a reminder system to intermittently prompt a user to consume liquid from the receptacle;
the sensor module further comprising an ambient light sensor for detecting an ambient light level around the drinking apparatus when on the support surface.

As noted above, advantageously, this may help to prevent disturbance of the user during periods of sleep, e.g. at night, when drinking is not required. The ambient light sensor may be housed within an interior of the sensor module and may be configured to detect the ambient light level by detecting signals transmitted through a face of the sensor module.

The reminder system comprises:
- one or more sensors configured to detect a drinking event associated with the user consuming liquid from the cavity;
- a timer configured to measure one or more predetermined time intervals;
- a speaker configured to generate one or more audio alerts and/or one or more lights configured to generate one or more visual alerts;
- a controller configured to generate a user alert if no drinking event is detected by the one or more sensors within a selected predetermined time interval as measured by the timer;
- a control interface; and
- a power source;

wherein the user alert may be one or more visual alerts generated by the one or more lights and/or one or more audio alerts generated by the speaker;
wherein the control interface may be configured to permit the user of the drinking apparatus to operate functions of the drinking apparatus.

The controller may be configured to provide a night-time mode in which generation of the user alert is prevented whenever the ambient light level is below a predetermined level as detected by the ambient light sensor. In the night-time mode the controller may be configured to activate the one or more lights when the ambient light level is below a predetermined level as detected by the ambient light sensor to thereby provide illumination for the user to locate the drinking apparatus. In the night-time mode the one or more lights may be activated in a mode pre-programmed to be one of: constant illumination, pulsing illumination or flashing illumination. The ambient light sensor may be a phototransistor.

The sensor module may further comprise:
- an audio recorder configured to allow recording of one or more audio messages by a user of the drinking apparatus - said one or more audio messages then being used as the one or more audio alerts.

The control interface may be configured to permit the user of the drinking apparatus to operate functions of the drinking apparatus including one or more of:
- switching the drinking apparatus on and off;
- the recording of the one or more audio messages;
- selection of one or more predetermined time intervals to be used by the controller for generation of the user alert;
- selection of the user alert from the one or more visual alerts and/or the one or more audio alerts.

For illustration purposes only, since the drinking apparatus of this invention is defined by all of the features of the appended independent claim 1, this disclosure furthermore relates to a drinking apparatus with the following features: There is provided a drinking apparatus comprising a receptacle and a sensor module;
the receptacle defining a cavity for holding a liquid for consumption;
wherein the sensor module can be coupled to the receptacle;
wherein the sensor module can be decoupled from the receptacle;
the drinking apparatus being able to freely stand on a support surface in a first orientation in which liquid can be retained in the cavity;
wherein the sensor module comprises one or more components including at least:
   - a tilt sensor configured to detect a tilting event associated with a user consuming liquid from the cavity;
   - a timer configured to measure one or more predetermined time intervals;
   - a speaker configured to generate one or more audio alerts and/or one or more lights configured to generate one or more visual alerts;
   - an audio recorder configured to allow recording of one or more audio messages by a user of the drinking apparatus - said one or more audio messages then being used as the one or more audio alerts;
   - a controller configured to generate a user alert if no tilting event is detected by the tilt sensor within a selected predetermined time interval as measured by the timer;
   - a control interface; and
   - a power source configured to power the one or more components;
wherein the user alert may be one or more visual alerts generated by the one or more lights and/or one or more audio alerts generated by the speaker;
wherein the control interface is configured to permit the user of the drinking apparatus to operate functions of the drinking apparatus including:
   - the recording of the one or more audio messages;
   - selection of one or more predetermined time intervals to be used by the controller for generation of the user alert;
   - selection of the user alert from the one or more visual alerts and/or the one or more audio alerts.

As in the above aspects, the user of the drinking apparatus may be a variety of classes of people. However, the present drinking apparatus provides particular benefits in a healthcare setting where a first class of user is a patient. A second class of user may be a care-giver that has responsibility for ensuring adequate hydration of the patient. Both the patient and the care-giver may be users of the same drinking apparatus.

The term "user alert" is used to refer to one or more alerts (which may differ in their characteristics from one another) that are generated to be received by the one or more users of the drinking apparatus. Different types of alert may be intended to be received and prompt action by different classes of user of the drinking apparatus. For example, one or more audio alerts may be intended to prompt action by the patient who is normally near the drinking apparatus and is thereby prompted to drink from the drinking apparatus when the one or more audio alerts are heard. For example, one or more visual alerts may be intended to prompt action by the care-giver (nurse, doctor, etc.) who may be further away from the drinking apparatus or only in the vicinity of the drinking apparatus periodically, e.g. when conducting rounds in a hospital ward setting.

Advantageously, the drinking apparatus of the present disclosure provides an improved means for prompting hydration of a user by allowing a combination of audio and visual alerts to be presented. Consequently different alerts may be targeted at different users (visual alerts detectable by a care-giver from long range) and audio alerts (which are localised for the patient so as not to disturb other patients nearby). Further, the audio alerts may be personalised, for example, by using the user's name, the voice of a family member, etc. which may improve the likelihood of the user responding positively to the instruction. Personised messages also reduce the likelihood of confusing alerts produced by different drinking apparatus that may be present in the same location.

The ability to use a plurality of audio messages (default or personalised) is advantageous compared to repetition of a single message in that it improves the effectiveness of the audio alerts. This is because the variety in the content of the messages makes it more likely that the user will take note and follow the prompting of the message. By contrast a single repeated message may actually aggravate a user making it less likely that a drink will be consumed when prompted. The present drinking apparatus also has the advantage of being able to refresh the personalised messages whenever needed so as to provide even more variety in the audio alerts.

The sensor module may form a base unit of the drinking apparatus which is coupled below the cavity such that in the first orientation the sensor module contacts the support surface.

When the sensor module is coupled to the receptacle the control interface may be inaccessible by the user; and optionally the control interface may comprise a plurality of buttons for inputting user commands which may be inaccessible when the sensor module is coupled to the receptacle.

The control interface may be configured to provide voice prompt feedback to the user.

The power source may comprise one or more batteries housed in a battery compartment, and when the sensor module is coupled to the receptacle the battery compartment may be inaccessible by the user.

The sensor module and the receptacle may comprise complementary connection means to permit coupling and decoupling of the sensor module and the receptacle; optionally wherein the complementary connection means comprises screw threads.

The receptacle may comprise a recess which receives at least a portion of the sensor module on coupling of the sensor module to the receptacle.

An annular seal may be provided between the sensor module and the receptacle to hinder or prevent ingress of water there between.

The tilt sensor may comprise a tilt switch and/or a gyroscope.

The drinking apparatus may further comprise an ambient light sensor for detecting an ambient light level around the drinking apparatus when on the support surface. The ambient light sensor may be housed within an interior of the sensor module and may be configured to detect the ambient light level by detecting signals transmitted through a face of the sensor module. The controller may be configured to provide a night-time mode in which generation of the user alert is prevented whenever the ambient light level is below a predetermined level as detected by the ambient light sensor. In the night-time mode the controller may be configured to activate the one or more lights when the ambient light level is below a predetermined level as detected by the ambient light sensor to thereby provide illumination for the user to locate the drinking apparatus. In the night-time mode the one or more lights may be activated in a mode pre-programmed to be one of: constant illumination, pulsing illumination or flashing illumination.

The sensor module may be at least splash-proof.

The audio recorder may comprise a microphone located within an interior of the sensor module.

The speaker may be located within an interior of the sensor module and may be configured to transmit sound waves via a face of the sensor module; and wherein optionally the speaker is an exciter speaker as described above in a previous aspect. Alternatively, the sensor module may be provided with an array of apertures for transmission of sound wave which are sealed by a membrane configured to be able to resonate as described above in a previous aspect.

The one or more lights may comprise one or more light-emitting diodes (LEDs). The one or more lights may be located within an interior of the sensor module and may be configured to transmit light through a face of the sensor module. The one or more visual alerts may comprise one or more patterns of intermittent flashing or pulsing of the one or more lights.

The controller may be configured to use one or more pre-recorded default audio alerts in the eventuality that the one or more audio messages are not recorded or selected. The control interface may be configured to permit the user of the drinking apparatus to select a volume of the one or more audio alerts. The controller may be configured to use the one or more audio messages sequentially if no tilting event is detected. The controller may be configured to repeat each audio alert at an increased volume. The one or more predetermined time intervals may include 20 minutes, 40 minutes and 60 minutes. The controller may be configured to reset the timer and restart measurement of the one or more predetermined time intervals whenever a tilting event is detected. The controller may be configured to interrupt the user alert, if being generated, whenever a tilting event is detected.

An interior face of the receptacle may comprise one or more filling-level markings, preferably annular rings.

The drinking apparatus may further comprise a lid that is detachably coupled to an open mouth of the receptacle. The lid may comprise a mouth port and/or a straw port.

The sensor module may be reusable and the receptacle may be disposable.

For illustration purposes only, since the method of this invention is defined by all of the features of the appended independent claim 13, this disclosure furthermore relates to a method of reminding a user to take a drink at intermittent times, comprising:
a) providing a drinking apparatus comprising a receptacle and a sensor module;
   the receptacle defining a cavity for holding a liquid for consumption;
   wherein the sensor module can be coupled to the receptacle when the drinking apparatus is being used for consumption of liquid from the cavity;
   wherein the sensor module can be decoupled from the receptacle;
   the drinking apparatus being able to freely stand on a support surface in a first orientation in which liquid can be retained in the cavity;
b) measuring one or more predetermined time intervals;
c) detecting a tilting event associated with a user consuming liquid from the cavity;
d) generating a user alert if no tilting event is detected within a selected predetermined time interval;
wherein the user alert is selected to be one or more visual alerts and/or one or more audio alerts; wherein said one or more audio alerts are one or more audio messages which have been recorded by a user of the apparatus.

For illustration purposes only, since the drinking apparatus of this invention is defined by all of the features of the appended independent claim 1, this disclosure furthermore relates to a drinking apparatus with the following features: There is provided a drinking system comprising a drinking apparatus and an external controller;
the drinking apparatus being as described in any of the above aspects and further comprising a receiver for receiving signals from the external controller;
the external controller comprising means for sending and/or receiving signals to and/or from the receiver of the sensor module, wherein the external controller is configured to carry out one or more of the following functions:
   - switching the drinking apparatus on and off;
   - the recording of the one or more audio messages;
   - selection of one or more predetermined time intervals to be used by the controller for generation of the user alert;
   - selection of the user alert from the one or more visual alerts and/or the one or more audio alerts; or
   - generating a user alert at the external controller if no tilting event is detected by the tilt sensor within a selected predetermined time interval as measured by the timer.

Advantageously, the drinking system permits one or more of the functions of the drinking apparatus to be programmed remote from the drinking apparatus.

The external controller may be a dedicated controller, for example a remote control, or a program or application running on an external device, for example a desktop computer, portable tablet, mobile phone, etc.

The signals between the external controller and the receiver of the sensor module may be any suitable signals, for example, infra-red, visible light, wi-fi, GSM, etc. The communication may be one way from the external controller to the receiver. Alternatively, the communication may comprise two-way communication of signals between the external controller and the receiver (in which case both the external controller and the receiver may function as transceivers).

The external controller may include means for recording one or more audio messages by the external controller which may then be transmitted to the receiver of the sensor module and stored as the one or more audio alerts. The external controller may include means for generating one or more audio alerts and/or one or more visual alerts as the user alert. A first external controller may be provided that is configured to program operation of the sensor module and a second external controller may be provided that is configured to generate the user alert.

### Brief Description of the Drawings:

The present disclosure will now be described, by way of example only, with reference to the accompanying drawings in which:
Figure 1 is a perspective view of a first embodiment of drinking apparatus according to the present disclosure including an optional lid;
Figure 2 is a perspective view of the drinking apparatus of Figure 1 with the lid removed;
Figure 3 is a perspective view of a receptacle of the drinking apparatus of Figure 2 with a sensor module removed;
Figure 4 is another view of the receptacle of Figure 3;
Figure 5 is a perspective view of the lid of the drinking apparatus of Figure 1;
Figure 6 is a cross-sectional view of the drinking apparatus of Figure 1 (not including the optional lid and omitting certain features for clarity);
Figure 7 is a perspective view of the sensor module of the drinking apparatus of Figure 1;
Figure 8 is a plan view of the sensor module of Figure 7;
Figure 9 is perspective view of the sensor module of Figure 7 with a battery compartment lid removed;
Figure 10 is a perspective view of a cup-shaped body of the sensor module of Figure 7;
Figure 11 is a perspective view of components of the sensor module of Figure 7;
Figure 12 is another perspective view of the components of Figure 11;
Figure 13 is a plan view from below of the components of Figure 11;
Figure 14 is a photograph of an example of a main PCB of a sensor module according to the present disclosure;
Figure 15 is a photograph of the opposite side of the main PCB of Figure 14;
Figure 16 is a photograph of a partially disassembled view of an example of a sensor module according to the present disclosure which incorporates the main PCB of Figure 14;
Figure 17 is a schematic representation of a first tilt sensor;
Figure 18 is a schematic representation of a second tilt sensor;
Figure 19 is a perspective view of a second embodiment of drinking apparatus according to the present disclosure;
Figure 20 is a side elevation of the drinking apparatus of Figure 19; and
Figure 21 is a schematic view of a third embodiment of drinking apparatus according to the present disclosure.

### Detailed description

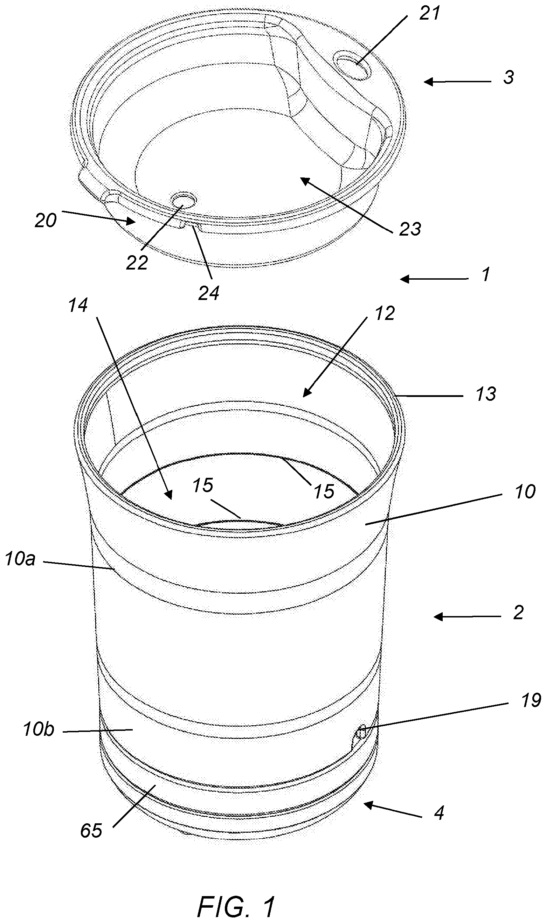

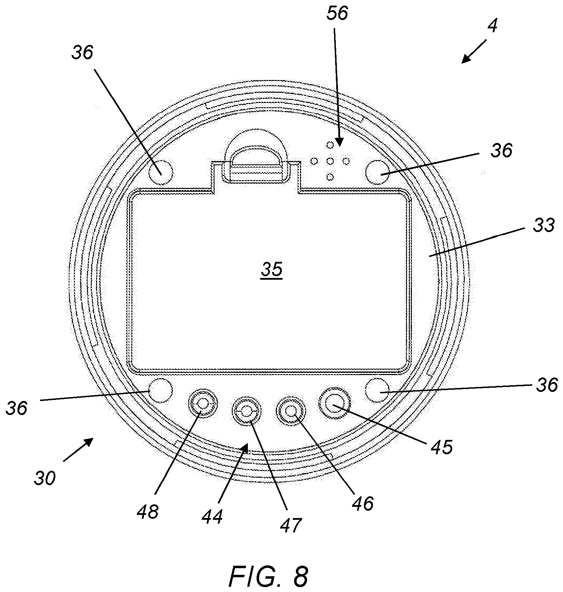

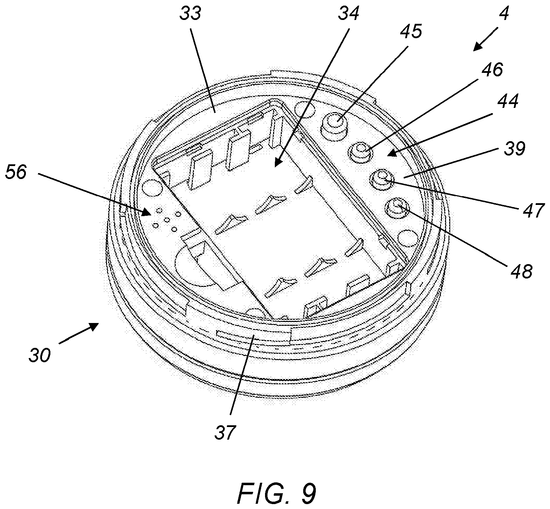

Figures 1 to 18 relate to a first embodiment of a drinking apparatus 1 according to the present disclosure. The drinking apparatus 1 comprises generally a receptacle 2 and a sensor module 4. In the current example the sensor module is illustrated and described in the form of a base unit that is coupled to a lower portion of the receptacle 2. However, the sensor module 4 may be configured to be coupled to other part or parts of the receptacle 2. The sensor module 4 may be coupled to the receptacle 2 to form the drinking apparatus 1 and decoupled from the receptacle 2 to allow operation of a control interface 43 of the sensor module 4. The control interface 43 permits a user of the drinking apparatus 1 to control generation of various functions of the drinking apparatus 1 as will be described below.

Optionally, the drinking apparatus may further comprise a lid 3, an example of which is shown in Figure 1 and will be described below. The lid 3 may be reusable or disposable.

As shown in Figures 1 to 4, the receptacle 2 may be generally cup-shaped. The receptacle 2 may comprise a side wall 10 which extends from a lower rim 18 to an upper rim 13. The side wall 10 may have a flared shape such that a diameter of the upper rim 13 is greater than a diameter of the lower rim 18.

A transverse wall 25 may be provided which extends generally perpendicularly to the side wall 10 and spans across the side wall 10. The transverse wall 25 may be located part-way along the side wall 10 to thereby define an upper section 9 of the receptacle 2 above the transverse wall 25 and a lower section 11 of the receptacle 2 below the transverse wall 25.

The upper section 9 of the receptacle 2 may form a cavity 14 which is suitable for holding a liquid for consumption. The cavity 14 may be bounded by an upper portion 10a of the side wall 10 and the transverse wall 25. An upper end of the cavity 14 may be an open mouth 12 of the receptacle 2 which may optionally receive the lid 3 as a push-fit (when used) to close or partially close the open mouth 12.

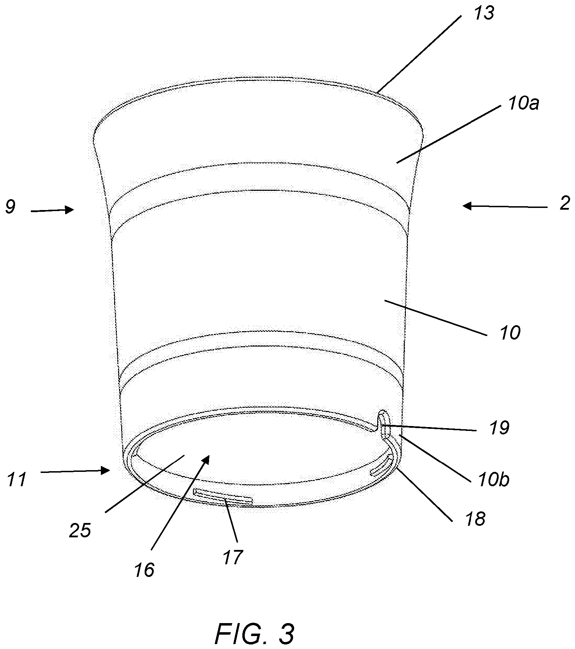

The lower section 11 of the receptacle 2 may form a recess 16 as most clearly shown in Figure 3. The recess 16 may be bounded by a lower portion 10b of the side wall 10 and the transverse wall 25. A lower end of the recess 16 may be open to receive the sensor module 4.

Thus, the transverse wall 25 may define a lower extremity of the cavity 14 on one side and an upper extremity of the recess 16 on the other side with the side wall 10 extending both above and below the level of the transverse wall 25.

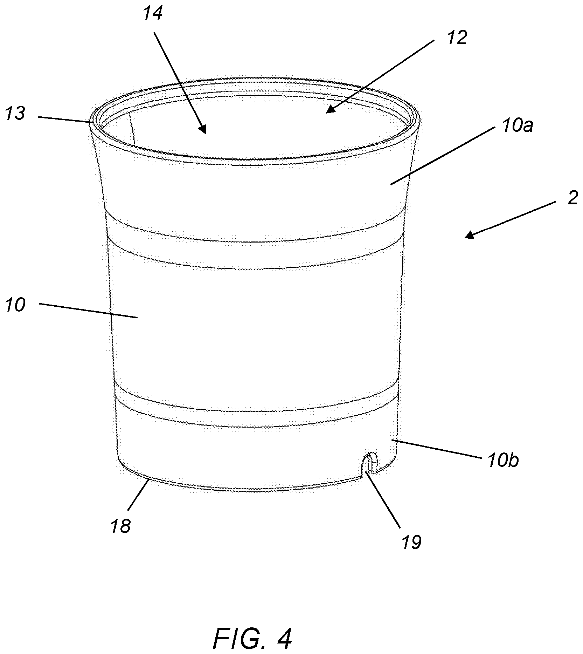

As shown in Figures 3 and 4, a drainage slot 19 may be provided in the side wall 10 within the lower section 11 which extends from the lower rim 18 upwardly to meet or approach the level of the transverse wall 25.

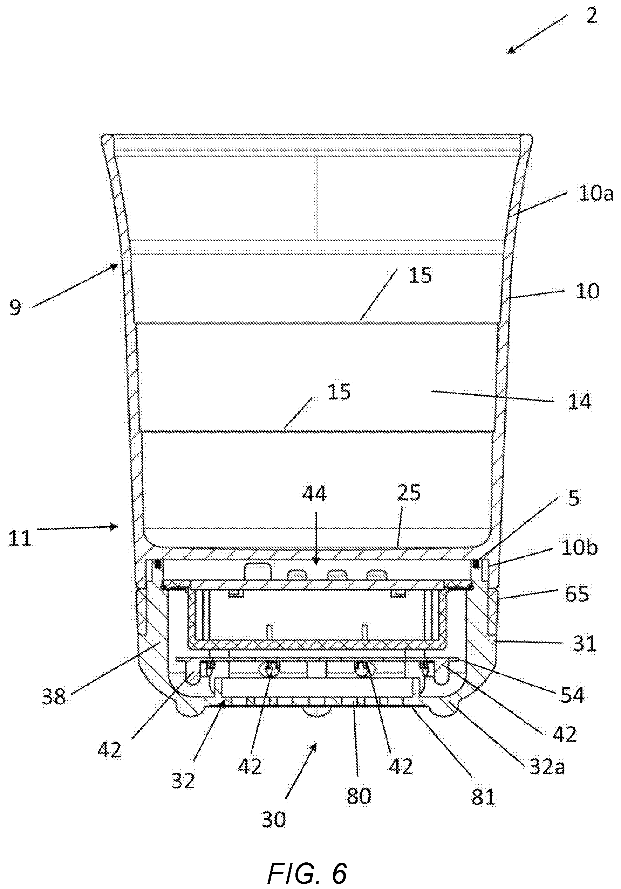

The transverse wall 25 may be planar as shown in Figure 6 or alternatively, and preferably, may be domed - in other words may have a convex surface facing the recess 16 and optionally also a concave surface facing the cavity 14. The drainage slot 19 allows for liquid left in the recess 16 after cleaning to drain more easily out of the recess 16 when the receptacle 2 is left to dry in an inverted or substantially inverted orientation with the open mouth 12 lowermost. The convexity of the surface of the transverse wall 25 within the recess 16 may aid drainage of liquid towards the drainage slot 19. More than one drainage slot 19 may be provided spaced around the circumference of the side wall 10.

The recess 16 provides a void space for allowing detachable coupling of the sensor module 4 to the receptacle 2. An internal face of the lower portion 10b of the side wall 10 within the recess 16 may be provided with a screw thread 17. In the illustrated example the screw thread 17 is provided as a number of discrete screw thread portions, preferably four screw thread portions 17, which are spaced apart around the periphery of the side wall 10.

An internal face of the upper portion 10a of the side wall 10 may be provided with one or more volume marking rings 15. In the illustrated example two volume marking rings 15 are provided, one demarcating a fill volume of the cavity 14 of 100ml and a second volume marking ring 15 demarcating a fill volume of 200ml.

The receptacle 2 may be formed from a choice of materials. Preferably the receptacle 2 is formed from a plastics material. The receptacle 2 is preferably formed as a single moulding but could be formed from multiple parts that are assembled together. More preferably the receptacle 2 is formed from a hard plastics material that is resistant to impacts and scratching. The material may be transparent or translucent. The material may preferably be free of Bisphenol A (BPA). One example of a suitable material is copolyester, for example Tritan^{®} plastic available from Eastman Chemical Company.

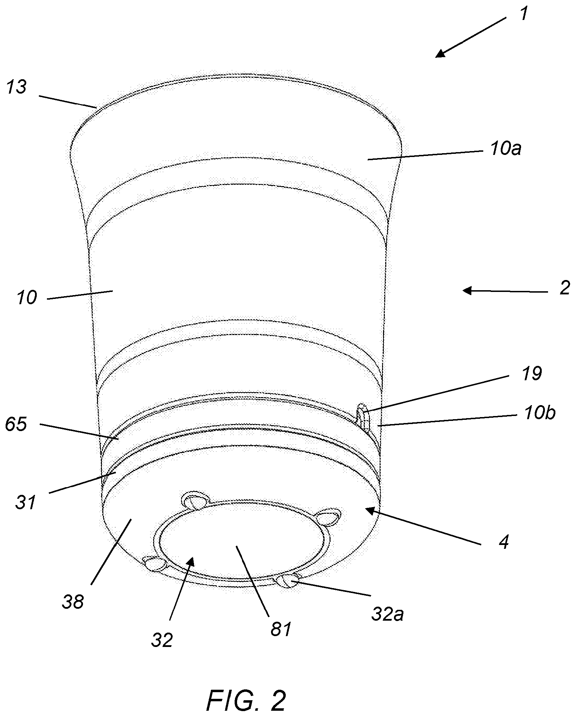

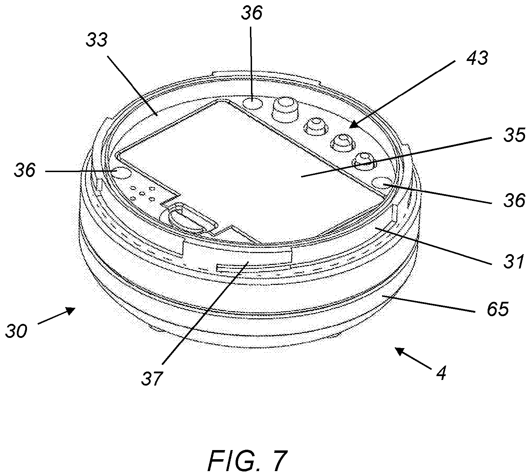

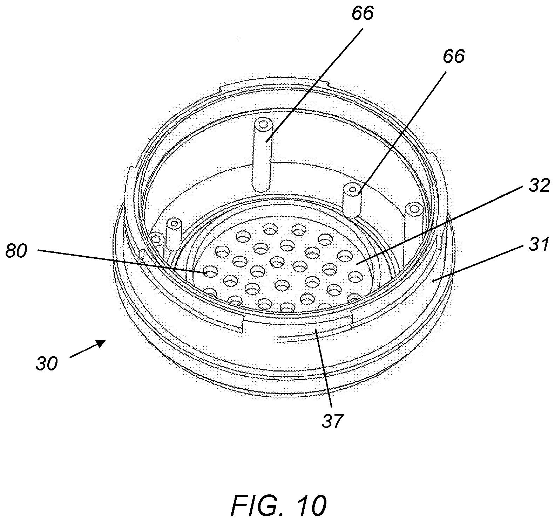

The sensor module 4 may be detachably coupled to the receptacle 2. The sensor module 4 and its components are shown in more detail in Figure 7 to 18. (For clarity some components are omitted and others are shown schematically in Figures 7 to 13). As shown in Figures 7 and 10, the sensor module 4 may comprise a generally cup-shaped body 30 which receives therein a chassis 33 to which are mounted various components of the sensor module 4. The cup-shaped body 30 may comprise a side wall 31 which extends upwardly from a bottom face 32 as shown in Figures 2 and 6. An optional décor ring 65 may be provided which is received over a portion of the side wall 31 as shown in Figure 7. If the décor ring 65 is not to be used then the side wall 31 may be sized to form a smooth transition into the lower portion 10b of the side wall 10.

The bottom face 32 may be generally flat and configured to be horizontal when the drinking apparatus 1 is placed on a horizontal and flat support surface. A plurality of protrusions 32a may be provided on the bottom face 32 to form stand-offs to raise the bottom face 32 off of the support surface. The bottom face 32 may a solid part of the cup-shaped body 30 that is without apertures. However, in the example illustrated in Figure 10, the portion of the cup-shaped body 30 forming the bottom is provided with an array of apertures 80 that pass through the cup-shaped housing. A separate thin membrane 81 is affixed to an exterior of the cup-shaped body 30 to cover the apertures 80 as shown in Figures 2 and 6. The membrane 81 may be set into a recessed portion of the exterior of the cup-shaped body 30 whose depth may be the same as the thickness of the membrane 81 so that on the exterior of the drinking apparatus 1 a smooth exterior surface is produced. The membrane 81 may be secured to the cup-shaped body 30 around the circumferential periphery only to allow the membrane 81 to resonate freely against the cup-shaped body 30 when driven by sound waves produced by a speaker located within the cup-shaped body 30, as will be described below. The membrane 81 may be formed from a plastics material that may be flexible.

The apertures 80 may improve the transmission of the sound waves out of the sensor module 4. The protrusions 32a may create a space between the membrane 81 and a support surface which provides space for the membrane 81 to resonate freely. The resonance of the membrane 81 may enhance the quality and/or audibility of the sound produced by the sensor module 4.

The side wall 31 may be configured to be vertical and extend at right angles to the bottom face 32. An interface between the side wall 31 and bottom face 32 may be provided with a radiused section 38 as shown in Figure 6.

As shown in Figure 7, the side wall 31 may be provided with a screw thread 37 which is engagable with the screw thread 17 provided on the receptacle 2. By this complementary connection means the sensor module 4 may be detachably coupled to the bottom portion of the receptacle 2 by screwing the sensor module 4 into the recess 16 of the receptacle 2. As shown in the cross section of Figure 6 and in the perspective view of Figure 2, once coupled to the receptacle 2, at least a portion of the sensor module 4 may be housed within the recess 16 of the receptacle 2. The décor ring 65 may be sized to provide a smooth surface interchange between the sensor module 4 and the receptacle 2. Thus, from the exterior, the user perceives a smooth interface between the side wall 10 of the receptacle 2, the décor ring 65 and then the side wall 31 which merges into the radiused section 38 and bottom face 32 of the sensor module 4.

The décor ring 65 may be chosen to have different colours and/or patterns and/or be provided with textural or pictorial identifiers to allow personalisation of the drinking apparatus 1. For example the décor ring 65 may be provided with the name of the user.

In the illustrated embodiment, the vertically extending side wall 31 of the sensor module 4 is only partially housed within the side wall 10 of the receptacle 2 which extends downwardly from the location of the transverse wall 25.

An annular seal 5, for example a gasket, may be provided between the cup-shaped body 30 and the receptacle 2. The annular seal 5 may be received in a rebate in an upper edge of the cup-shaped body 30 and may form a sealing interface against the transverse wall 25. Alternatively, or additionally, a seal may be provided that seals between the cup-shaped body 30 and the side wall 10. The annular seal 5 may hinder and preferably prevents ingress of water or other moisture into the recess 16 when the sensor module 4 is coupled to the receptacle 2.

As shown in Figure 6, with the sensor module 4 coupled to the receptacle 2, the drinking apparatus 1 comprises a stable integrated unit which may be freely stood on a support surface with the sensor module 4 in contact with the support surface and with the receptacle 2 orientated so as to be able to retain liquid within the cavity 14.

The cup-shaped body 30 of the sensor module 4 may be formed from a choice of materials. Preferably the cup-shaped body 30 is formed from a plastics material. The cup-shaped body 30 is preferably formed as a single moulding but could be formed from multiple parts that are assembled together. More preferably the cup-shaped body 30 is formed from a hard plastics material that is resistant to impacts and scratching. Preferably at least the bottom face 32 and the radiused section 38 are formed from a transparent or translucent material. Preferably the whole of the cup-shaped body 30 is formed from a transparent or translucent material. One example of a suitable material is copolyester, for example Tritan^{®} plastic available from Eastman Chemical Company.

The chassis 33 is located within the cup-shaped body 30 and is retained by means of a plurality of retaining screws 36 which pass through apertures in the chassis 33 into engagement with mounting points 66 of the cup-shaped body 30. The chassis 33 may be formed from a variety of materials including hard plastics and metals.

An upper portion 39 of the chassis 33 forms a top face of the sensor module 4. The chassis 33 further comprises a battery compartment 34 in the form of a rectangular-sectioned recess extending downwards from the upper portion 39 which is closed by a detachable battery compartment lid 35 which may be retained in the closed configuration by means of a clip or screw. The upper portion 39 the chassis 33 is further provided with four apertures through which project a plurality of interface buttons 44 which will be described further below. Further, the upper face of the chassis 33 is provided with sound transmission apertures 56 the use of which will, again, be described further below.

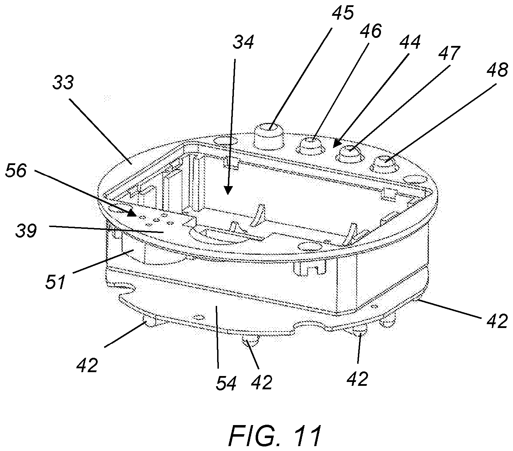

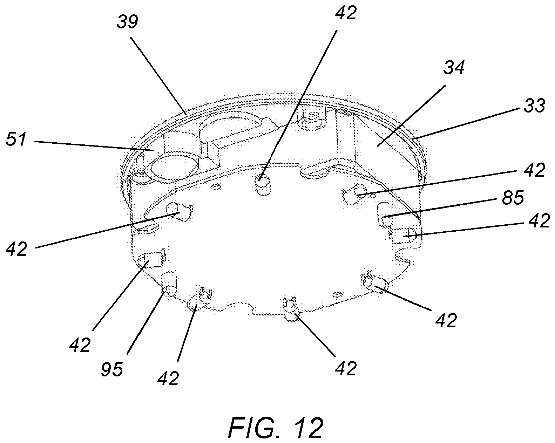

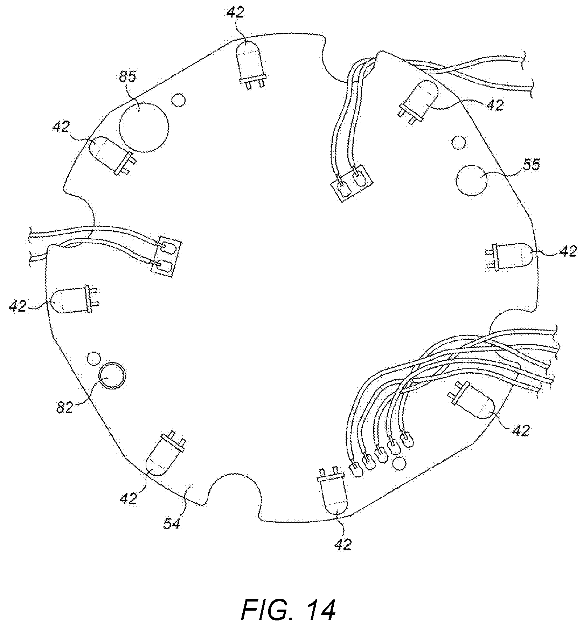

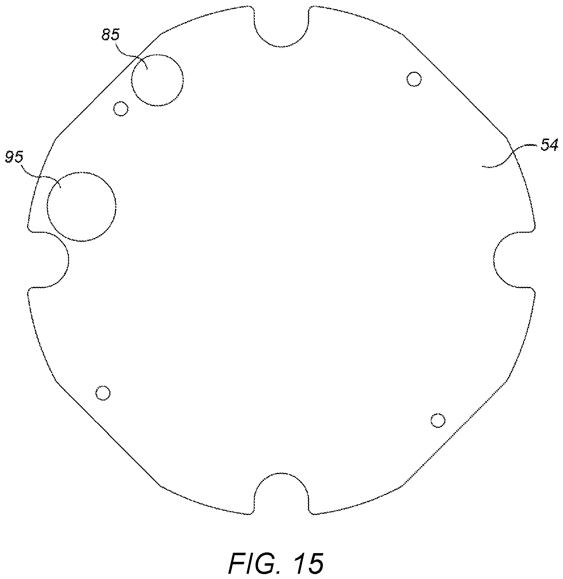

As shown in Figures 11 and 12, a main printed circuit board (PCB) 54 is provided which has mounted thereto a number of components of the sensor module 4. An example of a configuration of the main PCB 54 of the sensor module is shown in Figures 14 and 15. The main PCB 54 may be mounted to the cup-shaped body 30.

A microphone 51 is provided and may mounted to an underside of the upper portion 39 of the chassis 33. The microphone 51 may be connected by wires to the main PCB 54. Preferably the microphone is orientated to receive audio signals passing through the sound transmission apertures 56. Preferably the microphone 51 and sound transmission apertures 56 are aligned with one another.

A plurality of buttons 45-48, which may form the interface buttons 44, may be mounted to an upper PCB located between the main PCB 54 and the upper portion 39. The upper PCB may be mounted to the chassis 33. In the illustrated example, four buttons 45, 46, 47, and 48 are provided. The buttons 45-48 may be differently coloured to aid operation of the control interface 43 by a user. For example button 45 may be red, button 46 may be green, button 47 may be yellow and button 48 may be blue.

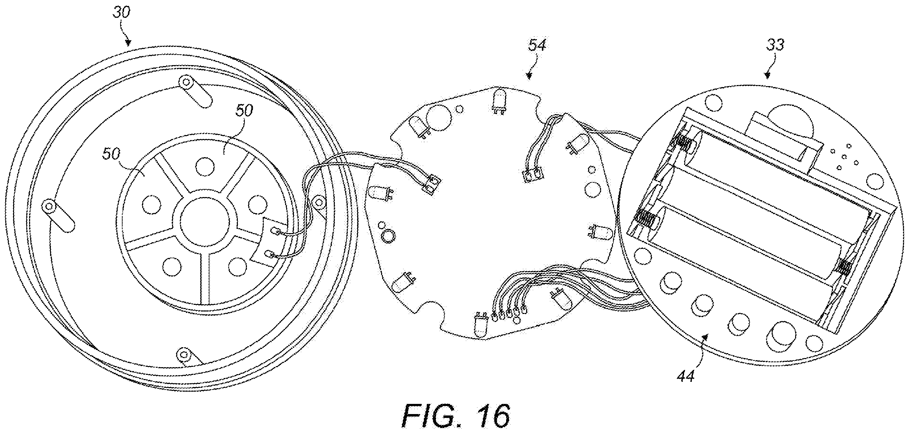

A speaker 50 may be connected to the main PCB 54 as shown in Figure 16. The speaker 50 is preferably orientated to transmit audio signals downwards towards the bottom face 32 of the sensor module 4 and/or sideways towards the side wall 31. The speaker 50 may be an exciter speaker. The speaker 50 may be configured to excite the bottom face 32. Alternatively, as illustrated the speaker 50 may be located above the array of apertures 80 and orientated to direct sound waves through the apertures 80 to resonate the membrane 81.

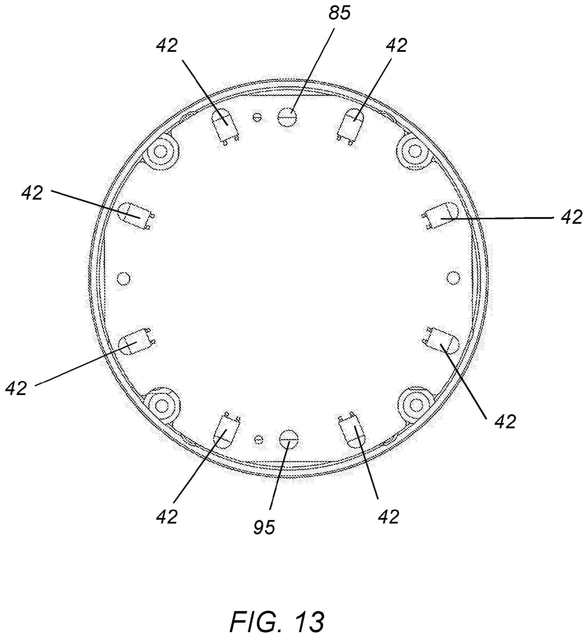

As shown in Figures 12 and 13, the main PCB 54 may also have mounted to it a plurality of lights and one or more sensors.

One or more light emitting diodes (LEDs) 42 are provided as a first functional group of lights. Separately, one or more further LEDs 82 are provided as a second functional group of lights. The LEDs 42 and LEDs 82 preferably differ in their colour. In one example the LEDs 42 are blue and the LEDs 82 are red. In one example eight blue LEDs 42 are provided and one or two red LEDs 82 are provided. In Figures 12 and 13 the red LEDs 82 are omitted for clarity. A red LED 82 is shown in the example of Figure 14. Figure 14 shows one red LED 82. A second red LED 82 may also be provided, preferably located diametrically opposite across the main PCB 54.

The LEDS 42, 82 may be located at or near a periphery of the main PCB 54 so as to be located adjacent or near the side wall 31 and/or bottom face 32 of the sensor module 4. As shown in Figure 6, the LEDs 42, 82 may preferably be located level with the radiused section 38 of the sensor module 4. The LEDs 42 may be equispaced around the periphery of the main PCB 54.

The LEDs 42 may form visual alert lights. The LEDs 82 may form one or more night-time mode location lights. The same LEDs 82 or another set of the LEDs may form one or more device notification lights. In one example the eight blue LEDs 42 may form eight visual alert lights that may be used to generate one or more visual alerts as will be described below. The one or two red LEDs 82 may form the one or more night-time mode location lights as will be described below. The same one or two red LEDs 82 may form the device notification light as will be described below.

The main PCB 54 may, in addition, have mounted on it an ambient light sensor 55, as shown in Figure 14, which is light-sensitive. The sensor may be in the form of a phototransistor. For example, a photodiode such as a PIN GaAs photodiode may be used. The ambient light sensor 55 may be attached to a periphery of the main PCB 54 so that it receives lights signals that have been transmitted through the transparent or translucent radiused section 38 and/or side wall 31 of the sensor module 4.

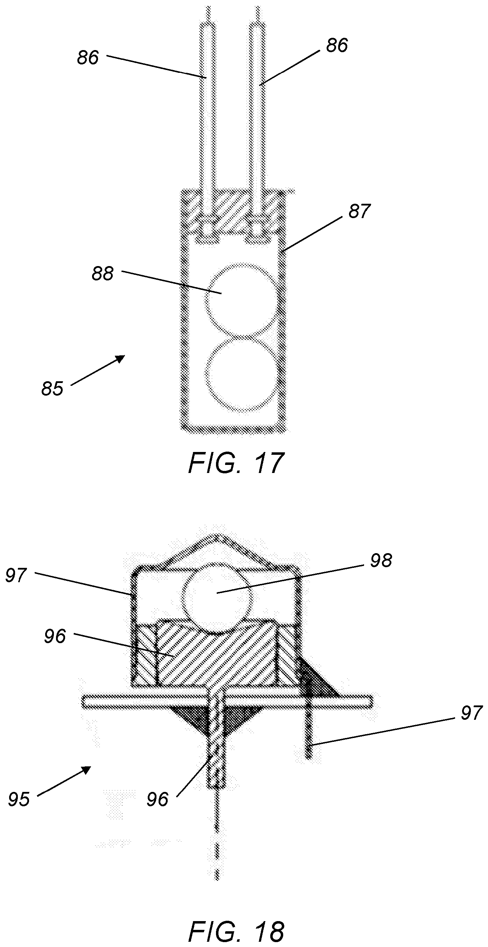

The main PCB 54 may have mounted on it a first tilt sensor 85, shown schematically in Figure 12 and shown in the example of Figures 14 and 15. The first tilt sensor may function as an 'inversion sensor' to detect a reorientation of the drinking apparatus 1 into a cleaning, orientation in which the mouth 12 of the receptacle 2 is angled downwards. Preferably, the first tilt sensor 85 is configured to detect when the receptacle 2 has been tilted away from a first, storage, orientation by greater than 90°, preferably greater than 100°, more preferably greater than 135°. (In a typical example, the angle of tilt will be measured from vertically upwards, i.e. a direction opposed to the pull of gravity such that an angle of tilt of greater than 90° would direct the mouth 12 of the receptacle 2 to below horizontal). The switching angle of the first tilt sensor 85 (i.e. the tilt angle at which the sensor changes from an open state to a closed state or vice versa) may be configured by appropriate selection of the switch design and geometry. One example of a suitable type of tilt sensor is a 'ball-in-can' tilt sensor, an example of which is shown schematically in Figure 17. In this type of sensor two switch contacts 86 are provided at one end of a housing 87 ('can') that are electrically isolated from one another. One or two electrically conductive balls 88, e.g. metal ball bearings) are movable within the housing 87 from a position out of contact with the switch contacts 86 (as shown in Figure 17) representing an open state of the switch to a position where the balls 88 bridge across the switch contacts 86 closing the switch. By appropriate configuration of the geometry of the housing 87, balls 88 and switch contacts 86 the switching angle of the tilt sensor can be changed.

Where the main PCB 54 is mounted laterally within the sensor module 4 (so that it will be orientated horizontally when the drinking apparatus 1 is stood on a horizontal support surface) the first tilt sensor 85 may be orientated vertically with the switch contacts 86 uppermost - so that the first tilt sensor 85 is in an open state when the drinking apparatus is stood on a support surface on its sensor module 4. Use of the first tilt sensor 85 will be described below.

The main PCB 54 may, in addition, have mounted on it a second tilt sensor 95, shown schematically in Figures 12 and shown in the example of Figure 15. The second tilt sensor 95 may function as a 'tilt sensor' to detect when a drink is consumed from the cavity 14 by a user. Preferably, the second tilt sensor 95 is configured to detect when the receptacle 2 has been tilted away from a first, storage, orientation by greater than 15°, preferably greater than 30°. (In a typical example, the angle of tilt will be measured from vertically upwards, i.e. a direction opposed to the pull of gravity). The switching angle of the second tilt sensor 95 may be configured by appropriate selection of the switch design and geometry. One example of a suitable type of tilt sensor is a 'ball-in-can' tilt sensor, an example of which is shown schematically in Figure 18. This example differs to some extent compared to the design of Figure 17. In this case, the two switching contacts are the housing 97 and a dished platen 96 that is electrically isolated from the housing 97. One electrically conductive ball 98 is provided that can rest in the dish of the dished platen 96 but be moved sideways into contact with the housing 97 when the sensor is tilted to close the switch. The sensor preferably has circular rotational symmetry about the dished platen 96 so that a tilt in any direction away from vertical can activate the sensor.

Where the main PCB 54 is mounted laterally within the sensor module 4 the second tilt sensor 95 may be orientated vertically with the dish of the dished platen 96 pointing upwards - so that the ball 98 rests in the dish when the drinking apparatus is stood on a support surface on its sensor module 4 so that the second tilt sensor 95 is in an open state. Use of the second tilt sensor 95 will be described below.

The main PCB 54 may also be provided with means for interconnecting and controlling the components of the sensor module 4. For example, a controller comprising one or more processors and one or more memories may be provided for storing and executing commands and programs for operating the sensor module 4. Means for conveying power to the components is also provided, for example in the form of batteries provided in the battery compartment 34 and suitable electrical connections on and between the PCBs.

The controller may also include a timer that is configured to measure time intervals under programmed control. The timer may provide for the timing of one or a plurality of time intervals.

The LEDs 42, 82, the first tilt sensor 85, the second tilt sensor 95 and the ambient light sensor 55 may all be housed within an interior of the sensor module 4 and not provided on an exterior face of the sensor module 4.

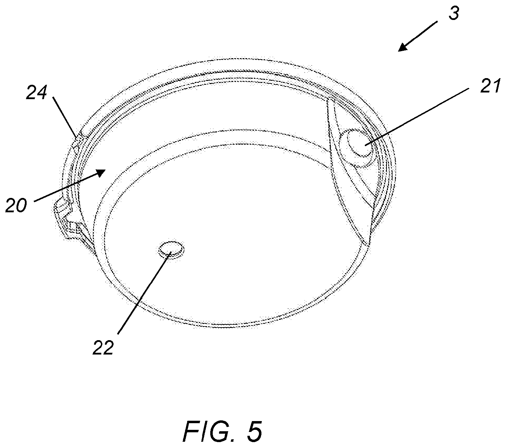

As shown in Figure 1, the open mouth 12 of the receptacle 2 may be fitted with the lid 3. The lid 3 may be a push fit into the open mouth 12. The lid 3 is shown in Figure 1 and also in Figure 5 and comprises a body 20 of a suitable shape to fit into the open mouth 12 of the receptacle 2. Preferably an upper face of the lid body 20 is provided with a dished recess 23 creating two levels of the external face of the lid 3. In an upper level, a mouth port 21 is provided for allowing a user to drink from the drinking apparatus 1 by applying their mouth directly to the mouth port 21. In the lower level, a straw port 22 is provided to allow insertion of a drinking straw. One or more drainage slots 24 may be provided in a rim of the lid 3 to allow better drainage of water from recesses of the lid 3 during cleaning.

The drinking apparatus 1 may make use of the features described above to provide a reminder system to intermittently prompt a user to consume liquid from the receptacle 2. In particular, the reminder system may use the second tilt sensor 95 to detect a drinking event associated with a user consuming liquid from the cavity 14 and may do so by detecting tilting of the drinking apparatus 1.

In use, the drinking apparatus 1 may function to generate a user alert if no tilting event is detected by the second tilt sensor 95 within one or more pre-determined time intervals as measured by the timer of the controller. The term "tilting event" is used to refer to a movement of the drinking apparatus 1 that is sufficient in extent for the second tilt sensor 95 to reach its switching angle and therefore for the tilt sensor to change from an open state to a closed state (or vice versa). If desired the controller may be configured so that only a tilting event lasting for a minimum duration is used to reset the timer and hence prevent (or halt) a user alert. For example, a minimum time interval of 2 or 3 seconds may be chosen. The term "user alert" is used to refer to one or more alerts (which may differ in their characteristics from one another) that are generated to be received by one or more users of the drinking apparatus 1. Different types of alert may be intended to be received and prompt action by different classes of user of the drinking apparatus 1. For example, one or more audio alerts may be intended to prompt action by a patient who is normally near the drinking apparatus 1 and is thereby prompted to drink from the drinking apparatus 1 when the one or more audio alerts are heard. For example, one or more visual alerts may be intended to prompt action by a care-giver who may be further away from the drinking apparatus 1 or only in the vicinity of the drinking apparatus 1 periodically.

Thus, the user alert may comprise one or more visual alerts and/or one or more audio alerts as will be described below. Different time intervals may be set for triggering different user alerts. For example a first time interval may be set for triggering the one or more visual alerts and a second time interval may be set for triggering the one or more audio alerts. For example, the one or more audio alerts may be triggered after 20 minutes and the one or more visual alerts may be triggered after 60 minutes.

Successive user alerts may be generated after the expiry of each successive pre-determined time interval if a tilting event is still not detected. For example, the pre-determined time interval for the one or more audio alerts may be selected as 20 minutes and one or more audio alerts would then be generated every 20 minutes until a tilting event was detected. Likewise, the pre-determined time interval for the one or more visual alerts may be selected as 60 minutes and one or more visual alerts would then be generated every 60 minutes until a tilting event was detected. In an alternative option, the one or more visual alerts once triggered may remain active until a tilting event is detected. Thus, in this configuration, the one or more visual alerts would not be repeated (although accompanying audio alerts may still be triggered). This arrangement may be preferred for alerting a care-giver who is only periodically near the drinking apparatus 1.

The type of user alert and the length of the time interval for each type of user alert may be set by the user by operation of the control interface 43. The control interface 43 may comprise the plurality of interface buttons 44 alone but preferably also includes voice prompts that are generated by the controller and transmitted via the speaker 50 in response to activation of the interface buttons 44. The voice prompts may confirm the current selection to the user. The interface buttons 44 may be configured to carry out a first action when given a short press (for example less than 1 second) and a second action when given a long press (for example 3 or 4 seconds or more).

Importantly, the control interface 43 may only be accessed and operated when the sensor module 4 is decoupled from the receptacle 2. Advantageously, this helps to prevent accidental activation of the control interface 43 when a user is drinking from the drinking apparatus 1. In addition, where the drinking apparatus 1 is for a patient who is under the supervision of a care-giver, the control interface 43 may be operated by the care-giver and the patient may be hindered from accidentally or deliberately altering the setting of the drinking apparatus 1 during use.

To use the drinking apparatus 1 the sensor module 4 must first be switched on. With the sensor module 4 decoupled from the receptacle 2, the user (or care-giver) may switch on the sensor module 4 by a long press on the red button 45. The device notification light in the form of the one or more red LEDs 82 may briefly flash once when the sensor module 4 is switched on, flash intermittently while the sensor module 4 is on, remain illuminated whenever the sensor module 4 is on, or provide a steady slow pulsing light whenever the sensor module 4 is on. In addition, when the sensor module 4 is first switched on it may provide an audio summary of the current settings of the module using the speaker 50.

The sensor module 4 may then be coupled to the receptacle 2 by means of the complementary connection means. A liquid for consumption, for example water or a isotonic beverage, is poured into the cavity 14. The volume marking rings 15 may be used to assist in filling a desired volume of liquid into the receptacle 2 which may be helpful if the beverage is being made up as a solution of a powdered component dissolved in a carrier fluid such as water.

If desired, the lid 3 may be fitted to the open mouth 12 and a straw inserted into the straw port 22 if required.

The drinking apparatus 1 is stood on a support surface, e.g. a bedside cabinet, when not being used. In this storage orientation the second tilt sensor 95 is not triggered. Typically the dished platen 96 will be horizontal, i.e. parallel to the support surface. The generation of user alerts is associated with detecting tilting of the drinking apparatus 1 away from the vertical. Tilting of the drinking apparatus 1 to at least the switching angle, e.g. 15° or 30°, will cause the second tilt sensor 95 to be closed and hence triggered (optionally after a minimum time threshold is exceeded). The triggering of the second tilt sensor 95 is detected by the controller which registers this as a tilting event.

A user alert is generated by the controller if no tilting event is detected by the second tilt sensor 95 within the one or more pre-determined time intervals as measured by the timer. (As noted above, the user alert may include either or both audio and visual alerts which may be set to be triggered after the same or different time intervals). If a tilting event occurs the counting of all measured time intervals by the timer is reset and timing recommences when the second tilt sensor 95 detects that the drinking apparatus 1 has been returned to vertical. Any user alert being generated may be interrupted if a tilting event occurs during the user alert.

A first type of user alert may be one or more visual alerts making use of the blue LEDs 42. Optionally the eight LEDs 42 may flash sequentially. They may flash in pairs sequentially. The two LEDs of each pair may be on opposed sides of the cup-shaped body 30. The order of illumination of the pairs of LEDs may be configured to create the impression of a moving set of lights. In one example a first pair of LEDs may be illuminated followed by a non-adjacent pair of LEDs, followed by an adjacent or non-adjacent pair of LEDs. For example, in the case of eight LEDs 42 which are equi-spaced (as shown in the example of Figure 14) these may be numbered 1 to 8 clockwise around the main PCB 54. An illumination pattern may then be numbers 1 and 5 illuminated first, followed by numbers 3 and 7, followed by numbers 2 and 6, followed by numbers 4 and 8. The pattern may then repeat. This may improve visibility of the alert irrespective of the position of the user relative to the sensor module 4.

This visual user alert may be programmed (when the sensor module 4 is decoupled) by short presses of the yellow button 47. In one example successive short presses of the yellow button 47 cycles the time interval for the one or more visual alerts between settings of 20 minutes, 40 minutes, 60 minutes and 'off' (when 'off' no visual alerts would be generated).

A second type of user alert may be one or more audio alerts making use of the speaker 50. This user alert may be programmed (when the sensor module 4 is decoupled) by short presses of the green button 46. In one example successive short presses of the green button 46 cycles the time interval for the one or more audio alerts between settings of 20 minutes, 40 minutes, 60 minutes and 'off' (when 'off' no audio alerts would be generated).

The first and/or second types of user alerts may be selected in any desired combination. In one example the visual alerts may be selected to generate after 20 minutes and the audio alerts may be switched off. In a second example the visual alerts may be selected to generate after 40 minutes and the audio alerts may selected to generate after 40 minutes as well. In a third example the audio alerts may be selected to generate after 20 minutes and the visual alerts may selected to generate after 60 minutes. Other combinations are of course possible as will be apparent to the reader.

The one or more visual alerts may be intermittent illumination (e.g. flashing) of the LEDs 42 or constant illumination of those LEDs or a steady slow pulsing light. The one or more visual alerts may be generated for a fixed period only or may be generated until a tilting event is detected. The one or more visual alerts may be visible to the user through the side wall 31 and radiused section 38 of the sensor module 4.

The one or more audio alerts may be any suitable audio signal. However, preferably the audio alerts are spoken messages conveying a command message, for example, "Have a drink", "Time to drink", etc. Preferably, a plurality of spoken messages may be provided which are generated cyclically.

The controller may be provided with one or more default spoken messages which are pre-recorded for use as the audio alerts, for example messages DEFAULT 1, DEFAULT 2 and DEFAULT 3. Message DEFAULT 1 would be played after expiry of a first pre-determined time interval, in this example 20 minutes, if no tilting event is detected. After a further 20 minutes message DEFAULT 2 would be played back if a tilting event is still not detected. After a further 20 minutes message DEFAULT 3 would be played back if a tilting event is still not detected. Thereafter, the pattern would repeat with messages DEFAULT 1, DEFAULT2 and DEFAULT 3 be played cyclically until a tilting event was detected.

Preferably, the sensor module 4 has the ability of allowing a user (e.g. a care-giver) to record one or more personalised messages, for example three messages, PERSONAL 1, PERSONAL 2 and PERSONAL 3 using the microphone 51. The personalised messages may then be used cyclically as the audio alerts instead of the default spoken messages in the same manner as just described for the default messages. Personalised messages may be recorded by a long press of the blue button 48. The user may check the content of the stored default or personalised messages by use of short presses of the blue button 48 which will sequentially playback the stored messages.

Therefore, for example three default spoken messages (DEFAULT 1, DEFAULT 2 and DEFAULT 3) may be stored in the memory of the controller and in addition three personalised messages (PERSONAL 1, PERSONAL 2 and PERSONAL 3) may be stored. If drinking apparatus 1 is reset to clear the personalised messages, the default spoken messages will still be stored and will be used as the audio alerts unless and until new personalised messages are recorded.

Each time an audio alert is generated it may optionally be repeated after a short delay, e.g. 5 to 7 seconds, to increase the likelihood that the user will notice the audio alert. The volume of the repeat may be set higher than for the original playback. The volume of each repeat may increase up to a set maximum volume.

The volume of the audio alerts may be set by use of the control interface 43. The volume may be programmed by use of short presses of the red button 45.

The drinking apparatus 1 may be provided with a night-time mode of operation in which generation of the user alert is prevented whenever the ambient light level is below a predetermined level as detected by the ambient light sensor 55. If desired the controller may be configured that only a change in ambient illumination lasting for a minimum duration is used to trigger starting or stopping of the night time mode. When the night-time mode is operational, the controller may be configured to activate the one or more red LEDs 82 to act as location lights to assist a user in finding the drinking apparatus in the dark. However, it is preferred that the level of illumination is kept low so as not to disturb a user's sleep. Therefore, preferably the maximum intensity of the location lights is kept to a predetermined level by configuration of the number or power of the LEDs used for the location lights. For example, this may be achieved by using only one of the red LEDs 82. In the night-time mode the one or more red LEDs 82 may be activated in a mode pre-programmed to be one of: constant illumination, pulsing illumination or flashing illumination. The night-time mode of operation may be switched on and off by long presses of the yellow button 47.

The LEDs 42 may also be used to provide a low battery level warning wherein they flash rapidly to alert a user that the batteries need replacing.

The drinking apparatus 1 may make use of the features described above to provide an alert system which is configured to generate a warning signal on detection of a reorientation of the drinking apparatus 1 into a cleaning orientation to warn a user against cleaning the drinking apparatus 1 while the sensor module 4 remains coupled to the receptacle 2. Such reorientation of the drinking apparatus 1 may be considered an inversion event. The term "inversion event" is used to refer to a movement of the drinking apparatus 1 that is sufficient in extent for the first tilt sensor 85 to reach its switching angle and therefore for the tilt sensor to change from an open state to a closed state (or vice versa).

The alert system may help to prevent a user exposing the sensor module 4 to a cleaning environment that may cause damage to the sensor module 4.

The alert system may be provided instead of the reminder system described above. However, preferably the alert system is provided as well as the reminder system. The alert system may make use of some or all of the same components as used by the reminder system. However, it is preferred that the alert system makes use of a dedicated sensor, for example the first tilt sensor 85 that is separate from the second tilt sensor 95. This allows the functional characteristics of both sensors to be optimised for their differing roles.

The alert system may be triggered when the mouth 12 of the receptacle 2 is angled downwards. In use the alert system may be triggered when the receptacle 2 has been tilted away from the a orientation by greater than 90°, preferably greater than 100°, more preferably greater than 135°. At the chosen angle the first tilt sensor 85 will be triggered which is detected by the controller of the sensor module and registered as the inversion event. Preferably in an inversion event the mouth 12 is angled downwards, which will typically mean directed below the horizontal plane. Preferably the degree of tilting in an inversion event is significantly greater than the degree of tilting in a tilting event. In this way the majority of tilting events will not be detected as an inversion event. However, this is not to exclude that an event may be both a tilting event and an inversion event.

In use, when the controller detects that an inversion event has occurred the blue LEDs 42 are triggered to generate a visual warning signal. Preferably the visual alert is generated immediately upon detection of the inversion event. The visual warning signal may be, for example, all eight LEDs42 flashing rapidly together. Preferably the visual warning signal for an inversion event is different to the visual alert triggered to prompt a user to consume a drink. Other alerts (audio, tactile) may be used as well or instead of a visual warning signal.

It will be noted that the alert system is configured to produce an alert on detection of a movement of the drinking apparatus 1 occurring whereas the reminder system is configured to produce an alert when a movement of the drinking apparatus has not occurred.

Cleaning of the drinking apparatus 1 in a dishwasher is an example of where an inversion event may occur. A user may be tempted to place the whole drinking apparatus 1 into the rack of a dishwasher. To do so the drinking apparatus 1 would be inverted (i.e. tilted or rotated) to point the mouth 12 downwards to allow for drainage of cleaning water from the cavity 14. Racks in dishwashers are typically angled slightly away from the horizontal. Thus placing the drinking apparatus 1 upside down in the rack would involve tilting the drinking apparatus through approximately 155 to 175°. Since this degree of tilting exceeds the switching angle of the first tilt sensor 85 an inversion event would be detected and the LEDs 42 would flash a warning alert to the user to prompt them to stop and remove the sensor module 4. Thereafter the receptacle 2 may be cleaned in the dishwasher on its own.

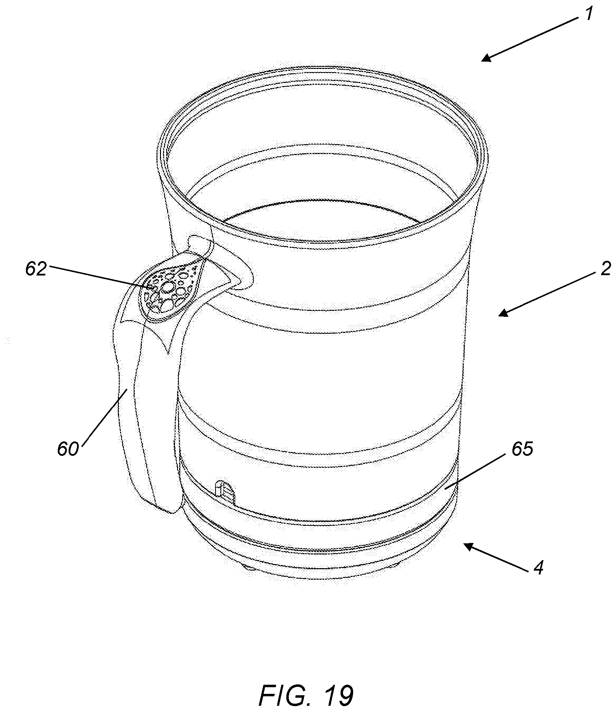

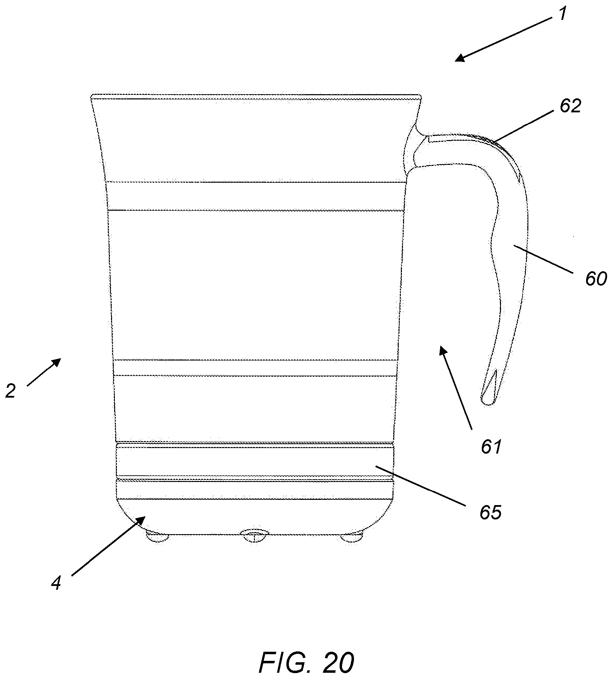

Figures 19 and 20 show a second embodiment of a drinking apparatus 1 according to the present disclosure. Many of the features of the second embodiment are the same as the first embodiment and will not be described further. In particular the same sensor module 4 may be used in the second embodiment of drinking apparatus 1 as in the first embodiment. Like reference numerals have been used for like components.

In the second embodiment the receptacle 2 takes the form of a handled cup and comprises a handle 60 which projects from the side wall 10 of the receptacle 2. Advantageously, the handle 60 may assist a user who has limited manual dexterity to grip the drinking apparatus 1. In particular, the handle 60 may be in a general L-shape providing an opening 61 facing towards the bottom to allow easy access for the fingers. The handle 60 may also be provided with a large grip area and a stabilising recess 62 on an upper part that may receive a user's thumb.

The present disclosure extends to provide a drinking system which may comprise a plurality of receptacles 2 and a single sensor module 4 that can be used with each receptacle 2. For example a receptacle 2 of the first embodiment (without a handle) and a receptacle 2 of the second embodiment (with a handle 60) may be supplied with a single sensor module 4 that is detachably couplable to either receptacle 2. This allows a user a choice of type of receptacle 2 to use. It may also allow the receptacle to form a disposable part of the drinking system and the sensor module 4 to form a reusable part of the drinking system. The drinking system may also comprise the optional lid 3. The lid 3 may form a disposable part of the drinking system.

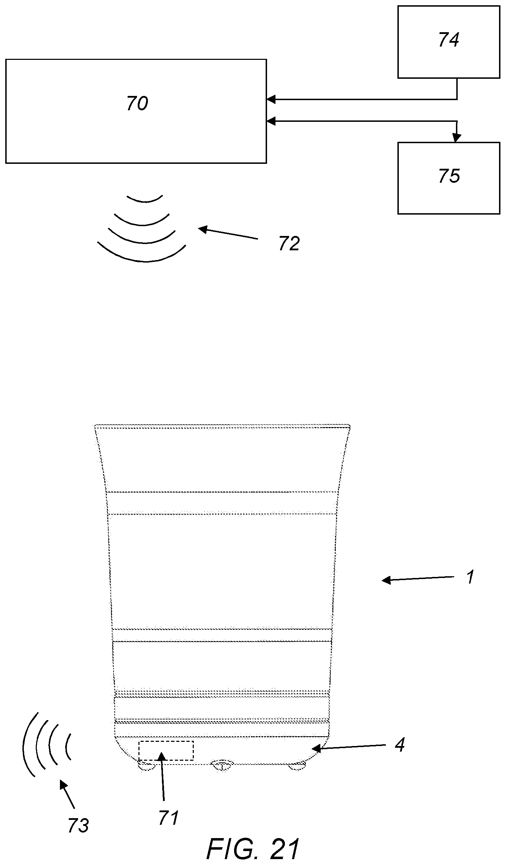

Figure 21 shows a third embodiment of a drinking apparatus 1 according to the present disclosure. Many of the features of the third embodiment are the same as the first embodiment and will not be described further. Like reference numerals have been used for like components.

In the third embodiment, the drinking apparatus 1 is configured to interact wirelessly with one or more external controllers 70 to permit programming of the operation of the sensor module 4 and/or the generation of a user alert remote from the drinking apparatus. Thus, the drinking apparatus 1 and the external controller(s) 70 together form a drinking system.

The drinking apparatus 1 may be as described above in any of the previous embodiments except for the following modifications of the sensor module 4. The main modification is that the sensor module 4 is provided with a receiver 71 for receiving signals from at least one external controller 70. The sensor module 4 may not be provided with a microphone in this embodiment.

The external controller 70 comprises means for sending signals to receiver 71 of the sensor module 4. This means may be a transmitter of known type. The signals 72 from the external controller 70 to the receiver 71 of the sensor module 4 may be any suitable signals, for example, infra-red, visible light, wi-fi, GSM, etc.

The communication may be one way from the external controller 70 to the receiver 71. Alternatively, the communication may comprise two-way communication of signals 72, 73 between the external controller 70 and the receiver 71 (in which case both the external controller 70 and the receiver 71 may function as transceivers). Further the receiver 71 may receive signals from one external controller 70 and send signals to another external controller 70.

The external controller 70 may be a dedicated controller, for example a remote control, or a program or application running on an external device, for example a desktop computer, portable tablet, mobile phone, etc.

The external controller 70 may be configured to permit operation of functions of the drinking apparatus 1 including one or more of:
- switching the drinking apparatus on and off;
- the recording of the one or more audio messages;
- selection of one or more predetermined time intervals to be used by the controller for generation of the user alert; or
- selection of the user alert from the one or more visual alerts and/or the one or more audio alerts.

Advantageously, the drinking system permits one or more of the functions of the drinking apparatus 1 to be programmed remote from the drinking apparatus 1.

The external controller 70 may include means 74 (e.g. a microphone) for recording one or more audio messages by the external controller 70 which may then be transmitted to the receiver 71 of the sensor module 4 and stored as the one or more audio alerts.

In addition, or alternatively, the external controller 70 may include means (e.g. a speaker and or a light) to generate a user alert if a tilting event is not detected within the measured time interval. The user alert generated at the external controller 70 may be in addition or instead of the user alert generated at the sensor module 4. This function may be useful in a care setting where the external controller 70 is located at, for example, a nursing station on a ward.

The external controller 70 may include a user interface 75 to permit a user to receive information (including but not limited to user alerts) and select commands.

The same external controller 70 may be used to generate user alerts as is used to program operation of the sensor module 4. Alternatively, a first external controller 70 may be provided that is dedicated for programming operation of the sensor module 4. A second external controller 70 may be provided by which the user alerts are generated. This may be advantageous in a ward setting where the sensor module 4 may be programmed by a care-giver stationed near the patient but the user alerts are generated at a centralised nursing station.

## Claims

1. A drinking apparatus (1) comprising a receptacle (2) and a sensor module (4);
the receptacle (2) defining a cavity (14) for holding a liquid for consumption which can be filled into the cavity (14) and drained from the cavity (14) through a mouth (12) of the receptacle (2);
wherein the sensor module (4) can be coupled to the receptacle (2);
wherein the sensor module (4) can also be decoupled from the receptacle (2) to allow the receptacle (2) to be cleaned separately from the sensor module (4);
the drinking apparatus (1) being able to freely stand on a support surface in a first, storage, orientation in which liquid can be retained in the cavity (14);
the sensor module (4) comprising an alert system which is able, when the sensor module (4) is coupled to the receptacle (2), to detect a reorientation of the drinking apparatus (1) into a second, cleaning, orientation in which the mouth (12) of the receptacle (2) is angled downwards; and
wherein the sensor module (4) further comprises a reminder system to intermittently prompt a user to consume liquid from the receptacle;
wherein the reminder system comprises:
- one or more sensors configured to detect a drinking event associated with a user consuming liquid from the cavity;
- a timer configured to measure one or more predetermined time intervals;
- a speaker (50) configured to generate one or more audio alerts and/or one or more lights configured to generate one or more visual alerts;
- a controller configured to generate a user alert if no drinking event is detected by the one or more sensors within a selected predetermined time interval as measured by the timer;
- a control interface (43); and
- a power source;
wherein the user alert is one or more visual alerts generated by the one or more lights and/or one or more audio alerts generated by the speaker (50);
wherein the control interface (43) is configured to permit the user of the drinking apparatus (1) to operate functions of the drinking apparatus (1); and
**characterized in that** the alert system being configured to generate a warning signal on detection of a reorientation of the drinking apparatus into the second, cleaning, orientation to warn a user against cleaning the drinking apparatus while the sensor module remains coupled to the receptacle; and **in that** the controller is configured to reset the timer and restart measurement of the one or more predetermined time intervals whenever a drinking event is detected.

2. A drinking apparatus (1) as claimed in claim 1, wherein:
in the second, cleaning, orientation the mouth (12) of the receptacle (2) is angled downwards to allow liquid to fully, or substantially fully, drain from the cavity (14) out of the mouth (12) of the receptacle (2); and/or
wherein in the second, cleaning, orientation the receptacle (2) has been tilted away from the first, storage, orientation by greater than 90°, preferably greater than 100°, more preferably greater than 135°; and/or
wherein in the second, cleaning, orientation the drinking apparatus is inverted, preferably so that the mouth of the receptacle is directed vertically, or nearly vertically downwards.

3. A drinking apparatus (1) as claimed in any preceding claim, wherein:
the alert system is configured to generate the warning signal immediately on detection of the reorientation of the drinking apparatus (1) into the second, cleaning, orientation; and/or
the alert system comprises a sensor configured to detect reorientation of the drinking apparatus into the second, cleaning, orientation.

4. A drinking apparatus (1) as claimed in any preceding claim, wherein the alert system comprises one or more lights configured to generate the warning signal in the form of a visual alert;
and optionally wherein the one or more lights comprise one or more light-emitting diodes (LEDs) (42);
and optionally wherein the one or more lights are located within an interior of the sensor module and are configured to transmit light through a face of the sensor module;
and optionally wherein the visual alert comprises one or more patterns of intermittent flashing or pulsing of the one or more lights.

5. A drinking apparatus (1) as claimed in any preceding claim, wherein the alert system comprises a speaker (50) configured to generate the warning signal in the form of an audible alert;
and optionally wherein the speaker (50) is located within an interior of the sensor module (4) and is configured to transmit sound waves via a face of the sensor module (4); and wherein optionally the speaker (50) is an exciter speaker.

6. A drinking apparatus (1) as claimed in any preceding claim, wherein:
at least a portion of the sensor module (4) is formed from a transparent or translucent material; and/or
the sensor module (4) is coupled to the receptacle below the cavity.

7. A drinking apparatus (1) as claimed in any preceding claim, wherein the sensor module (4) forms a base unit of the drinking apparatus (1);
and optionally wherein the drinking apparatus (1) is able to freely stand on the support surface with the base unit in contact with the support surface.

8. A drinking apparatus (1) as claimed in any preceding claim, wherein the one or more sensors configured to detect a drinking event associated with a user consuming liquid from the cavity comprises a sensor configured to detect tilting of the receptacle away from the vertical; and preferably the sensor is configured to detect tilting of the receptacle away from the vertical by greater than 15°, preferably greater than 30°;
and optionally wherein the sensor configured to detect tilting of the receptacle away from the vertical comprises a tilt sensor (85).

9. A drinking apparatus as claimed in claim 1 or claim 8, wherein the drinking apparatus (1) comprises the sensor configured to detect reorientation of the drinking apparatus into the second, cleaning, orientation and a separate one or more sensors configured to detect a drinking event associated with a user consuming liquid from the cavity.

10. A drinking apparatus (1) as claimed in any one of claims 1, 8 or 9, wherein the reminder system further comprises:
- an audio recorder configured to allow recording of one or more audio messages by a user of the drinking apparatus (1) - said one or more audio messages then being used as the one or more audio alerts;
and optionally the control interface (43) is configured to permit the user of the drinking apparatus (1) to record the one or more audio messages.

11. A drinking apparatus (1) as claimed in claim 10, wherein when the sensor module (4) is coupled to the receptacle (2) the control interface (43) is inaccessible by the user; and optionally the control interface (43) comprises a plurality of buttons (44) for inputting user commands which are inaccessible when the sensor module (4) is coupled to the receptacle (2);
and optionally wherein the receptacle (2) comprises a recess (16) which receives at least the control interface (43) of the sensor module (4) on coupling of the sensor module (4) to the receptacle (2) so as to render the control interface (43) inaccessible to the user while the sensor module (4) remains coupled to the receptacle (2);
and optionally wherein the power source comprises one or more batteries housed in a battery compartment (34), and when the sensor module (43) is coupled to the receptacle (2) the battery compartment (34) is inaccessible by the user.

12. A drinking system comprising a drinking apparatus (1) as claimed in any preceding claim and further comprising a second receptacle which can be detachably coupled to said sensor module in place of the receptacle (2);
and optionally wherein the second receptacle has a different shape and configuration to the receptacle (2).

13. A method of preventing damage to a drinking apparatus, wherein the drinking apparatus (1) is as claimed in any of claims 1 to 11, the method comprising the steps of:
using the alert system to detect a reorientation of the drinking apparatus (1) into the second, cleaning, orientation;
on said detection, generating a warning signal to warn a user against cleaning the drinking apparatus (1) while the sensor module (4) remains coupled to the receptacle.

14. A method as claimed in claim 13, comprising using the alert system to detect when the mouth of the receptacle (2) has been angled downwards to allow liquid to fully, or substantially fully, drain from the cavity (14) out of the mouth (12) of the receptacle (2), and then generating the warning signal;
and optionally comprising using the alert system to detect when the receptacle (2) has been tilted away from the first, storage, orientation by greater than 90°, preferably greater than 100°, more preferably greater than 135°, and then generating the warning signal;
and optionally comprising using the alert system to detect when the drinking apparatus (1) is inverted, preferably so that the mouth (12) of the receptacle (2) is directed vertically, or nearly vertically downwards, and then generating the warning signal.

15. A method as claimed in any of claims 13 to 14, comprising generating the warning signal immediately on detection of the reorientation of the drinking apparatus (1) into the second, cleaning, orientation.

## Patentansprüche

1. Trinkvorrichtung (1), umfassend einen Behälter (2) und ein Sensormodul (4);
wobei der Behälter (2) einen Hohlraum (14) zum Aufbewahren einer zu konsumierenden Flüssigkeit definiert, die in den Hohlraum (14) gefüllt und aus dem Hohlraum (14) durch eine Öffnung (12) des Behälters (2) abgelassen werden kann;
wobei das Sensormodul (4) mit dem Behälter (2) gekoppelt werden kann;
wobei das Sensormodul (4) auch von dem Behälter (2) entkoppelt werden kann, um eine Reinigung des Behälters (2) getrennt von dem Sensormodul (4) zu ermöglichen;
wobei die Trinkvorrichtung (1) in einer ersten Aufbewahrungsorientierung, in der Flüssigkeit in dem Hohlraum (14) zurückgehalten werden kann, frei auf einer Auflagefläche stehen kann;
das Sensormodul (4) ein Warnsystem umfasst, das in der Lage ist, wenn das Sensormodul (4) mit dem Behälter (2) gekoppelt ist, eine Umorientierung der Trinkvorrichtung (1) in eine zweite Reinigungsorientierung zu erkennen, in der die Öffnung (12) des Behälters (2) nach unten geneigt ist; und
wobei das Sensormodul (4) ferner ein Erinnerungssystem umfasst, um einen Benutzer intermittierend aufzufordern, Flüssigkeit aus dem Behälter zu konsumieren;
wobei das Erinnerungssystem umfasst:
- einen oder mehrere Sensoren, die konfiguriert sind, um ein mit einem Flüssigkeit aus dem Hohlraum konsumierenden Benutzer assoziiertes Trinkereignis zu erfassen;
- einen Zeitgeber, der konfiguriert ist, um ein oder mehrere vorgegebene Zeitintervalle zu messen;
- einen Lautsprecher (50), der konfiguriert ist, um einen oder mehrere akustische Warnsignale zu erzeugen, und/oder eine oder mehrere Leuchten, die konfiguriert sind, um ein oder mehrere visuelle Warnsignale zu erzeugen;
- eine Steuerung, die konfiguriert ist, um einen Benutzeralarm zu erzeugen, wenn von dem einen oder den mehreren Sensoren innerhalb eines ausgewählten vorgegebenen Zeitintervalls, das von dem Zeitgeber gemessen wird, kein Trinkereignis erkannt wird;
- eine Steuerschnittstelle (43); und
- eine Stromquelle;
wobei der Benutzeralarm ein oder mehrere visuelle Alarme, die durch die ein oder mehreren Leuchten erzeugt werden, und/oder ein oder mehrere akustische Alarme, die durch den Lautsprecher (50) erzeugt werden, umfasst;
wobei die Steuerschnittstelle (43) konfiguriert ist, um es dem Benutzer der Trinkvorrichtung (1) zu ermöglichen, Funktionen der Trinkvorrichtung (1) zu bedienen; und
**dadurch gekennzeichnet, dass** das Warnsystem konfiguriert ist, um ein Warnsignal bei der Erfassung einer Neuausrichtung der Trinkvorrichtung in die zweite Reinigungsausrichtung zu erzeugen, um einen Benutzer davor zu warnen, die Trinkvorrichtung zu reinigen, während das Sensormodul mit dem Behälter verbunden bleibt; und
dass die Steuerung konfiguriert ist, um den Zeitgeber zurückzusetzen und die Messung der ein oder mehreren vorgegebenen Zeitintervalle neu zu starten, wenn ein Trinkereignis erfasst wird.

2. Trinkvorrichtung (1) nach Anspruch 1, bei der
in der zweiten Reinigungsausrichtung die Öffnung (12) des Behälters (2) nach unten geneigt ist, damit die Flüssigkeit vollständig oder im Wesentlichen vollständig aus dem Hohlraum (14) über der Öffnung (12) des Behälters (2) abfließen kann; und / oder
- in der zweiten Reinigungsausrichtung der Behälter (2) um mehr als 90°, vorzugsweise mehr als 100°, stärker bevorzugt mehr als 135°, aus der ersten Aufbewahrungsausrichtung heraus gekippt ist; und/oder
in der zweiten Reinigungsausrichtung die Trinkvorrichtung umgedreht ist, vorzugsweise so, dass die Öffnung des Behälters vertikal oder nahezu vertikal nach unten gerichtet ist.

3. Trinkvorrichtung (1) nach einem der vorhergehenden Ansprüche, bei der:
das Warnsystem konfiguriert ist, um das Warnsignal unmittelbar bei Erkennung der Umorientierung der Trinkvorrichtung (1) in die zweite Reinigungsausrichtung zu erzeugen; und/oder
das Warnsystem einen Sensor umfasst, der konfiguriert ist, um die Umorientierung der Trinkvorrichtung in die zweite Reinigungsausrichtung zu erkennen.

4. Trinkvorrichtung (1) nach einem der vorhergehenden Ansprüche, bei der das Warnsystem ein oder mehrere Leuchten umfasst, die konfiguriert sind, um das Warnsignal in Form eines visuellen Alarms zu erzeugen;
und bei der optional die ein oder mehreren Leuchten ein oder mehrere Leuchtdioden (LEDs) (42) umfassen;
und optional, wobei die ein oder mehreren Leuchten innerhalb eines Innenraums des Sensormoduls angeordnet und konfiguriert sind, um Licht durch eine Fläche des Sensormoduls zu übertragen;
und optional, wobei der visuelle Alarm ein oder mehrere Muster von intermittierendem Blinken oder Pulsieren der ein oder mehreren Leuchten umfasst.

5. Trinkvorrichtung (1) nach einem der vorhergehenden Ansprüche, bei der das Alarmsystem einen Lautsprecher (50) umfasst, der konfiguriert ist, um das Warnsignal in Form eines hörbaren Alarms zu erzeugen;
und optional, bei der der Lautsprecher (50) in einem Innenraum des Sensormoduls (4) angeordnet und konfiguriert ist, um Schallwellen über eine Fläche des Sensormoduls (4) zu übertragen;
und optional, bei der der Lautsprecher (50) ein Erregerlautsprecher ist.

6. Trinkvorrichtung (1) nach einem der vorhergehenden Ansprüche, bei der:
mindestens ein Teil des Sensormoduls (4) aus einem transparenten oder transluzenten Material besteht; und/oder
das Sensormodul (4) mit dem Behälter unterhalb des Hohlraums verbunden ist.

7. Trinkvorrichtung (1) nach einem der vorhergehenden Ansprüche, bei der das Sensormodul (4) eine Basiseinheit der Trinkvorrichtung (1) bildet;
und optional, bei der die Trinkvorrichtung (1) in der Lage ist, mit der Basiseinheit in Kontakt mit der Auflagefläche frei auf der Auflagefläche zu stehen.

8. Trinkvorrichtung (1) nach einem der vorhergehenden Ansprüche, bei der die ein oder mehreren zum Erfassen eines mit einem Flüssigkeit aus dem Hohlraum konsumierenden Benutzer assoziierten Trinkereignisses konfigurierten Sensoren einen zum Erfassen einer Neigung des Behälters weg von der Vertikalen konfigurierten Sensor umfassen; und vorzugsweise der Sensor zum Erfassen einer Neigung des Behälters weg von der Vertikalen um mehr als 15°, vorzugsweise mehr als 30°, konfiguriert ist;
und optional, wobei der Sensor, der zum Erkennen einer Neigung der Aufnahme weg von der Vertikalen konfiguriert ist, einen Neigungssensor (85) umfasst.

9. Trinkvorrichtung nach Anspruch 1 oder 8, wobei die Trinkvorrichtung (1) den zum Erkennen einer Neuausrichtung der Trinkvorrichtung in die zweite Reinigungsausrichtung konfigurierten Sensor und einen oder mehrere separate, zum Erkennen eines mit einem Flüssigkeit aus dem Hohlraum konsumierenden Benutzer assoziierten Trinkereignisses konfigurierte Sensoren umfasst.

10. Trinkvorrichtung (1) nach einem der Ansprüche 1, 8 oder 9, bei der das Erinnerungssystem ferner umfasst:
- einen Audiorecorder, der konfiguriert ist, um die Aufzeichnung einer oder mehrerer Audionachrichten durch einen Benutzer der Trinkvorrichtung (1) zu ermöglichen, wobei die ein oder mehreren Audionachrichten dann als ein oder mehrere Audiowarnsignale verwendet werden;
und optional die Steuerschnittstelle (43) konfiguriert ist, um dem Benutzer der Trinkvorrichtung (1) zu ermöglichen, die ein oder mehreren Audionachrichten aufzuzeichnen.

11. Trinkvorrichtung (1) nach Anspruch 10, bei der, wenn das Sensormodul (4) mit dem Behälter (2) gekoppelt ist, die Steuerschnittstelle (43) für den Benutzer nicht zugänglich ist;
und optional die Steuerschnittstelle (43) eine Mehrzahl von Tasten (44) zur Eingabe von Benutzerbefehlen umfasst, die nicht zugänglich sind, wenn das Sensormodul (4) mit dem Behälter (2) gekoppelt ist;
und optional, bei der der Behälter (2) eine Aussparung (16) umfasst, die zumindest die Steuerschnittstelle (43) des Sensormoduls (4) beim Koppeln des Sensormoduls (4) mit dem Behälter (2) aufnimmt, um die Steuerschnittstelle (43) für den Benutzer unzugänglich zu machen, während das Sensormodul (4) mit dem Behälter (2) gekoppelt bleibt;
und optional, bei der die Stromquelle eine oder mehrere Batterien umfasst, die in einem Batteriefach (34) untergebracht sind, und wenn das Sensormodul (43) mit dem Behälter (2) verbunden ist, das Batteriefach (34) für den Benutzer unzugänglich ist.

12. Trinksystem, umfassend eine Trinkvorrichtung (1) nach einem der vorhergehenden Ansprüche und ferner umfassend einen zweiten Behälter, der anstelle des Behälters (2) abnehmbar mit dem Sensormodul verbunden werden kann;
wobei optional der zweite Behälter eine andere Form und Konfiguration als der Behälter (2) aufweist.

13. Verfahren zum Verhindern von Schäden an einer Trinkvorrichtung, wobei die Trinkvorrichtung (1) wie in einem der Ansprüche 1 bis 11 beansprucht ist, wobei das Verfahren die folgenden Schritte umfasst:
Verwenden des Warnsystems, um eine Neuausrichtung der Trinkvorrichtung (1) in die zweite, die Reinigungsausrichtung zu erfassen;
bei dieser Erfassung ein Warnsignal zu erzeugen, um einen Benutzer davor zu warnen, die Trinkvorrichtung (1) zu reinigen, während das Sensormodul (4) mit dem Behälter verbunden bleibt.

14. Verfahren nach Anspruch 13, umfassend die Verwendung des Warnsystems, um zu erkennen, wenn die Öffnung des Behälters (2) nach unten geneigt wurde, um Flüssigkeit vollständig oder im Wesentlichen vollständig aus dem Hohlraum (14) aus der Öffnung (12) des Behälters (2) ablaufen zu lassen, und dann das Warnsignal zu erzeugen;
und optional umfassend die Verwendung des Warnsystems, um zu erkennen, wenn der Behälter (2) um mehr als 90°, vorzugsweise mehr als 100°, stärker bevorzugt mehr als 135°, aus der ersten Lagerungsausrichtung herausgekippt wurde, und dann Erzeugen des Warnsignals;
und optional umfassend die Verwendung des Warnsystems, um zu erkennen, wenn die Trinkvorrichtung (1) umgedreht wird, vorzugsweise so, dass die Öffnung (12) des Behälters (2) vertikal oder nahezu vertikal nach unten gerichtet ist, und dann Erzeugen des Warnsignals.

15. Verfahren nach einem der Ansprüche 13 bis 14, umfassend die Erzeugung des Warnsignals unmittelbar bei Erkennung der Umorientierung der Trinkvorrichtung (1) in die zweite Reinigungs-Ausrichtung.

## Revendications

1. Appareil de consommation de boisson (1) comprenant un récipient (2) et un module capteur (4) ;
le récipient (2) définissant une cavité (14) destinée à contenir un liquide pour consommation qui peut être versé dans la cavité (14) et évacué de la cavité (14) à travers une embouchure (12) du récipient (2) ;
dans lequel le module capteur (4) peut être couplé au récipient (2) ;
dans lequel le module capteur (4) peut également être découplé du récipient (2) pour permettre au récipient (2) d'être nettoyé séparément du module capteur (4) ;
l'appareil de consommation de boisson (1) pouvant se tenir librement debout sur une surface de support dans une première orientation de stockage dans laquelle du liquide peut être retenu dans la cavité (14) ;
le module capteur (4) comprenant un système d'alerte qui est capable, lorsque le module capteur (4) est couplé au récipient (2), de détecter une réorientation de l'appareil de consommation de boisson (1) dans une seconde orientation de nettoyage dans laquelle l'embouchure (12) du récipient (2) est penchée vers le bas ; et
dans lequel le module capteur (4) comprend en outre un système de rappel pour inciter par intermittence un utilisateur à consommer un liquide à partir du récipient ;
dans lequel le système de rappel comprend :
- un ou plusieurs capteurs configurés pour détecter un événement de consommation de boisson associé à un utilisateur consommant un liquide provenant de la cavité ;
- une minuterie configurée pour mesurer un ou plusieurs intervalles de temps prédéterminés ;
- un haut-parleur (50) configuré pour générer une ou plusieurs alertes audio et/ou une ou plusieurs lumières configurées pour générer une ou plusieurs alertes visuelles ;
- un dispositif de commande configuré pour générer une alerte utilisateur si aucun événement de boisson n'est détecté par les un ou plusieurs capteurs dans un intervalle de temps prédéterminé sélectionné tel que mesuré par la minuterie ;
- une interface de commande (43) ; et
- une source d'alimentation en énergie ;
dans lequel l'alerte utilisateur est une ou plusieurs alertes visuelles générées par les une ou plusieurs lumières et/ou une ou plusieurs alertes audio générées par le haut-parleur (50) ;
dans lequel l'interface de commande (43) est configurée pour permettre à l'utilisateur de l'appareil de consommation de boisson (1) d'activer des fonctions de l'appareil de consommation de boisson (1) ; et
**caractérisé en ce que** le système d'alerte est configuré pour générer un signal d'avertissement lors de la détection d'une réorientation de l'appareil de consommation de boisson dans la seconde orientation de nettoyage pour avertir un utilisateur de ne pas nettoyer l'appareil de consommation de boisson alors que le module capteur reste couplé au récipient ; et **en ce que**
le dispositif de commande est configuré pour réinitialiser la minuterie et redémarrer une mesure des un ou plusieurs intervalles de temps prédéterminés à chaque fois qu'un événement de consommation de boisson est détecté.

2. Appareil de consommation de boisson (1) selon la revendication 1, dans lequel :
dans la seconde orientation de nettoyage, l'embouchure (12) du récipient (2) est penchée vers le bas pour permettre au liquide de s'évacuer complètement, ou sensiblement complètement, de la cavité (14) hors de l'embouchure (12) du récipient (2) ; et/ou
dans lequel, dans la seconde orientation de nettoyage, le récipient (2) a été incliné par rapport à la première orientation de stockage de plus de 90°, de préférence de plus de 100°, de manière davantage préférée de plus de 135° ; et/ou
dans lequel, dans la seconde orientation de nettoyage, l'appareil de consommation de boisson est retourné, de préférence de sorte que l'embouchure du récipient soit dirigée verticalement, ou presque verticalement, vers le bas.

3. Appareil de consommation de boisson (1) selon une quelconque revendication précédente, dans lequel :
le système d'alerte est configuré pour générer le signal d'avertissement immédiatement lors de la détection de la réorientation de l'appareil de consommation de boisson (1) dans la seconde orientation de nettoyage ; et/ou
le système d'alerte comprend un capteur configuré pour détecter la réorientation de l'appareil de consommation de boisson dans la seconde orientation de nettoyage.

4. Appareil de consommation de boisson (1) selon une quelconque revendication précédente, dans lequel le système d'alerte comprend une ou plusieurs lumières configurées pour générer le signal d'avertissement sous la forme d'une alerte visuelle ;
et facultativement dans lequel les une ou plusieurs lumières comprennent une ou plusieurs diodes électroluminescentes (LED) (42) ;
et facultativement dans lequel les une ou plusieurs lumières sont situées dans un intérieur du module capteur et sont configurées pour transmettre de la lumière à travers une face du module capteur ;
et facultativement dans lequel l'alerte visuelle comprend un ou plusieurs motifs de clignotement ou d'impulsion intermittent des une ou plusieurs lumières.

5. Appareil de consommation de boisson (1) selon une quelconque revendication précédente, dans lequel le système d'alerte comprend un haut-parleur (50) configuré pour générer le signal d'avertissement sous la forme d'une alerte sonore ;
et facultativement dans lequel le haut-parleur (50) est situé dans un intérieur du module capteur (4) et est configuré pour transmettre des ondes sonores via une face du module capteur (4) ; et dans lequel facultativement le haut-parleur (50) est un haut-parleur à excitateur.

6. Appareil de consommation de boisson (1) selon une quelconque revendication précédente, dans lequel :
au moins une partie du module capteur (4) est formée d'un matériau transparent ou translucide ; et/ou
le module capteur (4) est couplé au récipient en dessous de la cavité.

7. Appareil de consommation de boisson (1) selon une quelconque revendication précédente, dans lequel le module capteur (4) forme une unité de base de l'appareil de consommation de boisson (1) ;
et facultativement dans lequel l'appareil de consommation de boisson (1) peut se tenir librement debout sur la surface de support avec l'unité de base en contact avec la surface de support.

8. Appareil de consommation de boisson (1) selon une quelconque revendication précédente, dans lequel les un ou plusieurs capteurs configurés pour détecter un événement de consommation de boisson associé à un utilisateur consommant du liquide provenant de la cavité comprennent un capteur configuré pour détecter une inclinaison du récipient par rapport à la verticale ; et de préférence, le capteur est configuré pour détecter une inclinaison du récipient par rapport à la verticale de plus de 15°, de préférence de plus de 30° ;
et facultativement, dans lequel le capteur configuré pour détecter une inclinaison du récipient par rapport à la verticale comprend un capteur d'inclinaison (85).

9. Appareil de consommation de boisson selon la revendication 1 ou la revendication 8, dans lequel l'appareil de consommation de boisson (1) comprend le capteur configuré pour détecter une réorientation de l'appareil de consommation de boisson dans la seconde orientation de nettoyage et un ou plusieurs capteurs séparés configurés pour détecter un événement de consommation de boisson associé à un utilisateur consommant du liquide provenant de la cavité.

10. Appareil de consommation de boisson (1) selon l'une quelconque des revendications 1, 8 ou 9, dans lequel le système de rappel comprend en outre :
- un enregistreur audio configuré pour permettre l'enregistrement d'un ou plusieurs messages audio par un utilisateur de l'appareil de consommation de boisson (1) - lesdits un ou plusieurs messages audio étant alors utilisés en tant que les une ou plusieurs alertes audio ;
et facultativement l'interface de commande (43) est configurée pour permettre à l'utilisateur de l'appareil de consommation de boisson (1) d'enregistrer les un ou plusieurs messages audio.

11. Appareil de consommation de boisson (1) selon la revendication 10, dans lequel, lorsque le module capteur (4) est couplé au récipient (2), l'interface de commande (43) est inaccessible à l'utilisateur ; et facultativement l'interface de commande (43) comprend une pluralité de boutons (44) pour saisir des instructions utilisateur qui sont inaccessibles lorsque le module capteur (4) est couplé au récipient (2) ;
et facultativement dans lequel le récipient (2) comprend un évidement (16) qui reçoit au moins l'interface de commande (43) du module capteur (4) lors du couplage du module capteur (4) au récipient (2) de manière à rendre l'interface de commande (43) inaccessible à l'utilisateur alors que le module capteur (4) reste couplé au récipient (2) ;
et facultativement dans lequel la source d'alimentation en énergie comprend une ou plusieurs batteries logées dans un compartiment de batterie (34), et lorsque le module capteur (43) est couplé au récipient (2), le compartiment de batterie (34) est inaccessible à l'utilisateur.

12. Système de consommation de boisson comprenant un appareil de consommation de boisson (1) selon l'une quelconque des revendications précédentes et comprenant en outre un second récipient qui peut être couplé de manière amovible audit module capteur à la place du récipient (2) ;
et facultativement dans lequel le second récipient a une forme et une configuration différentes de celles du récipient (2).

13. Procédé de prévention de dommages sur un appareil de consommation de boisson, dans lequel l'appareil de consommation de boisson (1) est selon l'une quelconque des revendications 1 à 11, le procédé comprenant les étapes consistant à :
utiliser le système d'alerte pour détecter une réorientation de l'appareil de consommation de boisson (1) dans la seconde orientation de nettoyage ;
lors de ladite détection, générer un signal d'avertissement pour avertir un utilisateur de ne pas nettoyer l'appareil de consommation de boisson (1) alors que le module capteur (4) reste couplé au récipient.

14. Procédé selon la revendication 13, comprenant l'utilisation du système d'alerte pour détecter lorsque l'embouchure du récipient (2) a été penchée vers le bas pour permettre au liquide de s'évacuer complètement, ou sensiblement complètement, de la cavité (14) hors de l'embouchure (12) du récipient (2), puis la génération du signal d'avertissement ;
et comprenant facultativement l'utilisation du système d'alerte pour détecter lorsque le récipient (2) a été incliné par rapport à la première orientation de stockage de plus de 90°, de préférence de plus de 100°, de manière davantage préférée de plus de 135°, puis la génération du signal d'avertissement ;
et comprenant facultativement l'utilisation du système d'alerte pour détecter lorsque l'appareil de consommation de boisson (1) est retourné, de préférence de telle sorte que l'embouchure (12) du récipient (2) est dirigée verticalement, ou presque verticalement, vers le bas, puis la génération du signal d'avertissement.

15. Procédé selon l'une quelconque des revendications 13 à 14, comprenant la génération du signal d'avertissement immédiatement lors de la détection de la réorientation de l'appareil de consommation de boisson (1) dans la seconde orientation de nettoyage.
